# EUROPÄISCHE PATENTSCHRIFT

(11) **EP 3 837 233 B1**
(45) Veröffentlichungstag und Bekanntmachung des Hinweises auf die Patenterteilung: **26.06.2024**
(21) Anmeldenummer: 19752514.0
(22) Anmeldetag: 14.08.2019
(51) Int. Cl.: C07C 29/76, C07C 35/12

(54) **VERFAHREN ZUR HERSTELLUNG VON GEGEN VERBACKUNG STABILISIERTEN MENTHOLPARTIKELN SOWIE LAGERSTABILE MENTHOLPARTIKEL UND IHRE VERWENDUNG**
METHOD FOR PRODUCING MENTHOL PARTICLES STABILIZED AGAINST CAKING, AND STORAGE-STABLE MENTHOL PARTICLES AND USE THEREOF
PROCÉDÉ DE PRODUCTION DE PARTICULES DE MENTHOL STABILISÉES CONTRE L'AGGLOMÉRATION ET PARTICULES DE MENTHOL STABLE AU STOCKAGE ET LEUR UTILISATION

(30) Priorität: 16.08.2018 EP 18189338
(43) Veröffentlichungstag der Anmeldung: 23.06.2021
(73) Patentinhaber: BASF SE, 67056 Ludwigshafen am Rhein (DE)
(72) Erfinder: WLOCH, Sebastian, 67056 Ludwigshafen (DE); HEYDRICH, Gunnar, 67056 Ludwigshafen (DE); TEBBEN, Gerd, 67056 Ludwigshafen (DE); RAULS, Matthias, 67056 Ludwigshafen (DE)
(74) Vertreter: Meissner Bolte Partnerschaft mbB
(86) Internationale Anmeldenummer: PCT/EP2019/071770
(87) Internationale Veröffentlichungsnummer: WO 2020/035515

(56) Entgegenhaltungen:
- EP-A1- 3 059 009
- WO-A1-2008/152009
- "Verfahren zur Herstellung von gegen Verbackung stabilisierten Mentholpartikel sowie lagerstabile Mentholpartikel und ihre Verwendung", IP.COM JOURNAL, IP.COM INC., WEST HENRIETTA, NY, US, 6. September 2018 (2018-09-06), XP013179901, ISSN: 1533-0001

## Beschreibung

### GEBIET DER ERFINDUNG

Die vorliegende Erfindung betrifft ein Verfahren zur Herstellung von gegen Verbackung stabilisierten Mentholpartikeln, dadurch gekennzeichnet, dass man Mentholpartikel im Anschluss an die Formgebung für mindestens 7 Tage bei einer Temperatur von 0 bis 30°C lagert und anschließend den Mentholpartikeln ein Mindestmaß an mechanischer Energie zuführt. Die vorliegende Erfindung betrifft weiterhin lagerstabile Mentholpartikel sowie die Verwendung dieser Mentholpartikel in Gebrauchs- und Verbrauchsgüter aller Art.

### STAND DER TECHNIK

Menthol ist ein natürlich vorkommender Wirkstoff, der in Pharmazie, Kosmetik und Lebensmittelindustrie breite Anwendung findet. Menthol bewirkt beim Kontakt mit Schleimhäuten, insbesondere der Mundschleimhaut, einen kühlenden Effekt. In natürlichen Quellen, beispielsweise dem Pfefferminzöl, kommt Menthol in Form von vier diastereomeren Enantiomerenpaaren vor, von denen nur die Hauptkomponente, das (-)-Menthol oder L-Menthol die gewünschten geschmacklichen und sonstigen sensorischen Eigenschaften hat.

Von L-Menthol ist seit langem bekannt, dass es in vier verschiedenen Kristallmodifikationen erstarren kann, die bei gleicher chemischer Zusammensetzung unterschiedliche physikalische Eigenschaften aufweisen, wie bereits in J. Am. Chem. Soc., Vol. 39 (8), 1917, S. 1515 bis 1525 beschrieben. So liegen insbesondere die Schmelzpunkte dieser verschiedenen Modifikationen zwischen 33°C bis 43°C wie in Archiv der Pharmazie, 307 (7), 1974, S. 497 bis 503 beschrieben. Der Schmelzpunkt der stabilen alpha-Modifikation liegt demgemäß bei 42 bis 43°C.

Wegen dieser Lage der Schmelzpunkte kann L-Menthol an den Zwischen- oder Endverbraucher sowohl als in beheizten Behältern flüssig gehaltene Schmelze als auch in Form von Kristallen oder anderen erstarrten Formkörpern abgegeben werden. Generell weisen alle Feststoffe, die, wie L-Menthol, einen Schmelzpunkt nur knapp oberhalb der Umgebungstemperatur haben, eine starke Neigung auf, zu verbacken und zu verklumpen. Die Verarbeitung solcherma-ßen verbackenen Materials ist aber mit erheblichem, unerwünschten Zusatzaufwand verbunden. Soll nun reines L-Menthol, d.h. nicht mit Hilfsmitteln wie z.B. Trennmitteln behandeltes Menthol als Feststoff verkauft werden, muss durch die Art der Formgebung sichergestellt werden, dass das Produkt den Zwischen- oder Endverbraucher in rieselfähiger Form erreicht.

Kommerziell ist Menthol beispielsweise in Form großer Kristallnadeln verfügbar, die bei einer Länge von 0,5 bis 3 cm eine Dicke von 1 bis 3 mm aufweisen. Sie werden traditionell in kleinen Mengen aus natürlich gewonnenem Pfefferminzöl gezüchtet, in dem das Öl in Trögen oder Wannen über viele Tage in Kühlhäusern zur Kristallisation gebracht wird. Diese Kristallnadeln weisen nur bei geringer Schütthöhe eine gute Rieselfähigkeit auf, verbacken aber zusehends bei erhöhter Last und/oder erhöhter Temperatur. Diese Kristallnadeln weisen außerdem herstellungsbedingt immer Reste des Öls, aus dem sie gewonnen wurden, auf. Der technische Aufwand für die Kristallisation, Abtrennung und Reinigung der Kristallnadeln und die geringe Raum-Zeit-Ausbeute eines solchen langwierigen Verfahrens machen es unattraktiv.

Die DE 25 30 481 betrifft eine Vorrichtung zum Kristallisieren von Substanzen, insbesondere von optisch aktiven Mentholen, die unter Kristallisationsbedingungen grobe nadel- und balkenförmige Kristalle bilden. Das diskontinuierlich durchzuführende Kristallisationsverfahren wird unter Einsatz eines besonderen Rührwerkes durchgeführt, das ein Zusammenbacken der Kristalle in der Kristallsuspension verhindert. Das Wertprodukt wird abschließend durch eine Zentrifuge isoliert und in einem Trockner getrocknet.

Die US 3,023,253 und die US 3,064,311 (Bain) beschreiben geschupptes L-Menthol sowie ein Verfahren zur Herstellung derartiger Schuppen durch Aufbringen einer Schmelze von L-Menthol auf einer gekühlten Tauchwalze. Falls gewünscht kann die Mentholschmelze zwischen ein Paar gegenläufig rotierender, gekühlter Walzen eingebracht werden. Der auf der Tauchwalze ankristallisierte Mentholfilm wird nachbehandelt, indem er durch Wärmeeintrag getempert und durch Aufbringen zusätzlichen Menthols verstärkt wird. Beide Nachbehandlungen werden simultan mit einer Auftragswalze erreicht. Die so erhaltenen Schuppen weisen zunächst eine gute Rieselfähigkeit auf. Nach längerer Lagerung tritt jedoch Verbackung ein.

Das Prinzip der weiteren Vergröberung der Primärpartikel durch Kompaktierung wird auch in der WO 03/101924 (Symrise) betreffend kompaktiertes Menthol in Form von Menthol-Presslingen sowie ein Verfahren zur Herstellung derselben beschrieben. Hier wird aber nicht auf die Wirkung der Partikelgröße allein abgehoben, sondern darauf, dass die Primärpartikel in einer spezifischen Kristallmodifikation vorliegen müssen. Durch Verpressen von Kristallen, die aus einer Lösungskristallisation oder einer Kühlwalzenverschuppung gewonnen wurden, lassen sich Kompaktate erhalten.

WO 2008/152009 (BASF) beschreibt ein Verfahren zur Herstellung von L-Menthol in fester Form, speziell in Form von Schuppen, durch Inkontaktbringen einer L-Menthol-Schmelze mit zwei voneinander beabstandeten gekühlten Oberflächen sowie das nach dem genannten Verfahren erhältliche L-Menthol in fester Form, sowie dessen Verwendung zur Einarbeitung in Gebrauchs- und Verbrauchsgüter aller Art.

WO 2016/016154 (Symrise) beschreibt ein Verfahren zur Herstellung von festen Kühlstoffen, bei dem an eine vorgegrätzte, d.h. mit Impfkristallen versehene Schmelze von Mentholverbindungen durch gleichmäßige Vertropfung auf eine vorgekühlte Fläche gibt. Die so erhaltenen Mentholpartikel in Pastillenform weisen eine gewölbte und eine flache Seite auf und haben einen Durchmesser von etwa 1 bis etwa 20 mm.

EP 3 059 009 A1 (Symrise) beschreibt ein Verfahren zur verbackungsfreien Lagerung von festen Kühlstoffe, bei dem man diese in Normverpackungen mit einem Fassungsvermögen von maximal 25 I mit den Maßgaben abfüllt, dass die Verpackungen bis maximal zu 50 Vol% gefüllt sind und die Füllmenge ein Gewicht von 10 kg nicht übersteigt.

WO2006/097427 (Symrise) beschreibt Mentholhaltige Feststoffzusammensetzungen umfassend oder bestehend aus einer festen Mentholkomponente sowie einer festen Siliciumdioxidkomponente.

WO2007/071512 (Symrise) beschreibt sphärische Mentholpartikel sowie ein Verfahren zur Herstellung von sphärischen Mentholpartikeln, bei dem eine Menthol Schmelze in Wasser vertropft wird.

WO 2016/034481 (Sandvik) beschreibt ein Verfahren und eine Vorrichtung zur Herstellung von L-Menthol Pastillen, bei dem Schmelzetropfen einer Mentholschmelze über einen Tropfenformer auf einem Kühlband abgelegt und dort verfestigt werden.

In Anbetracht des genannten Standes der Technik bestand die Aufgabe der vorliegenden Erfindung in der Bereitstellung von lagerstabilen Mentholpartikeln sowie eines Verfahrens zur Herstellung von gegen Verbackung stabilisierten Mentholpartikel. Das Verfahren sollte sich insbesondere in technischem Maßstab mit möglichst geringem apparativem Aufwand und hohem Durchsatz betreiben lassen; die erhaltenen, gegen Verbackung stabilisierten Mentholpartikel sollten rieselfähig sein und insbesondere über einen längeren Zeitraum nur in geringem Ausmaß zur Verbackung neigen. Daneben soll sich das Verfahren besonders wirtschaftlich, d.h. kostengünstig betreiben lassen. Weiterhin sollte sich dieses Verfahren für Mentholpartikel der verschiedensten Arten der Formgebungen eignen.

### BESCHREIBUNG DER ERFINDUNG

Ein Gegenstand der vorliegenden Erfindung ist ein Verfahren zur Herstellung von gegen Verbackung stabilisierten Mentholpartikeln, dadurch gekennzeichnet, dass man Mentholpartikel im Anschluss an die Formgebung für mindestens 7 Tage bei einer Temperatur von 0 bis 30°C lagert und anschließend den Mentholpartikeln mindestens so viel mechanische Energie zuführt, wie sie erhalten, wenn man
- 20 kg dieser Mentholpartikel
- in einen Beutel (B) aus einer 0,12 mm dicken Polyethylen-Folie mit den Maßen Länge L(B) von 660 mm und Breite B(B) von 690 mm füllt, den Beutel verschließt;
- diesen Beutel in eine quaderförmige Kiste (K) aus zweiwelliger Wellpappe mit den Innenmaßen Länge L(K) von 385 mm, Breite B(K) von 320 mm und Höhe H(K) von 450 mm und einer Wellpappendicke von 6 mm verpackt, und
- diese Kiste einmalig aus einer Höhe von 1,0 m planparallel mit der von L(K) und H(K) gebildeten Seite auf eine unelastische Oberfläche fallen lässt.

Das als Verbackung oder Verklumpung bezeichnete Phänomen ist wahrscheinlich im Wesentlichen auf die Zusammenlagerung von Partikeln zu Agglomeraten zurückzuführen. Überraschenderweise wurde gefunden, dass mit dem erfindungsgemäßen Verfahren erhältliche Mentholpartikel gegen Verbackung stabilisiert sind. Dies bedeutet insbesondere, dass die Anzahl der Partikel in einem Kollektiv bei Lagerung von mehr als 18 Wochen bei 20 °C weniger stark abnimmt als Anzahl der Partikel in einem Vergleichskollektiv, welches nicht dem erfindungsgemäßen Verfahren unterzogen wurde. Die so erhältlichen Mentholpartikel sind auch nach Lagerung von mehr als 18 Wochen bei 20°C frei-rieselfähig und können so beispielsweise direkt, ohne weiteren Energieaufwand, für die Herstellung von Gebrauchs- und Verbrauchgütern, wie beispielsweise kosmetische und pharmazeutische Zubereitungen oder Nahrungsmitteln und Süßwaren eingesetzt werden.

Das erfindungsmäße Verfahren ist durch 2 Schritte gekennzeichnet. In einem ersten Schritt werden die als Ausgangsprodukte für das erfindungsgemäße Verfahren einzusetzenden Mentholpartikel im Anschluss an die Formgebung für mindestens 7 Tage bei einer Temperatur von 0 bis 30 °C gelagert. Anschließend wird in einem zweiten Schritt den gelagerten Mentholpartikeln mechanische Energie zugeführt. Dabei wird den Mentholpartikeln mindestens so viel mechanische Energie zugeführt, wie sie erhalten, wenn man
- 20 kg dieser Mentholpartikel
- in einen Beutel (B) aus einer 0,12 mm dicken Polyethylen-Folie mit den Maßen Länge L(B) von 660 mm und Breite B(B) von 690 mm füllt, den Beutel verschließt,
- diesen Beutel in eine quaderförmige Kiste (K) aus zweiwelliger Wellpappe mit den Innenmaßen Länge L(K) von 385 mm, Breite B(K) von 320 mm und Höhe H(K) von 450 mm und einer Wellpappendicke von 6 mm verpackt, und
- diese Kiste einmalig aus einer Höhe von 1,0 m planparallel mit der von L(K) und H(K) gebildeten Seite auf eine unelastische Oberfläche fallen lässt.

Die Lagerung im Anschluss an die Formgebung kann bei Temperaturen von 0 bis 30°C, vorzugsweise bei Temperaturen von 5 bis 25°C, insbesondere von 10 bis 23°C, vorzugsweise von 12 bis 20°C erfolgen. Die Lagerung erfolgt für mindestens 7 Tage. Die Lagerung kann beispielsweise für 10 Tage bei 20°C oder für 21 Tage bei 18°C erfolgen.

Die Lagerzeit beginnt mit Abschluss der Formgebung der einzusetzenden Mentholpartikel. Dieser ist der Zeitpunkt, zu dem die einzusetzenden Mentholpartikel ihre gewünschte Form, Größe und/oder Größenverteilung erhalten haben.

Die Lagerdauer ist, sofern die 7 Tage erreicht sind, unkritisch. Daher sind Lagerzeiten von beispielsweise 14 Tagen, 21 Tagen, aber auch mehreren Monaten, wie beispielsweise 1, 2, 6 oder 12 Monaten oder Jahren denkbar. Üblicherweise erfolgt die Lagerung für mindestens 7 Tage bis zu 3 Monaten, insbesondere bis zu 4, insbesondere bis zu 6 Monaten.

Die Lagerbedingungen außer der Temperatur und Mindestlagerdauer sind unkritisch. Die Lagerung kann in üblichen Verpackungseinheiten, wie beispielsweise Beuteln, Säcken, Kisten, Fässern oder Kombinationen daraus erfolgen. Bei der Abfüllung der Mentholpartikel in die Verpackungseinheiten ist vorteilhafterweise darauf zu achten, dass die Taupunkttemperatur nicht unterschritten wird, damit sich kein Kondenswasser in der Verpackungseinheit bildet. Vorteilhafterweise sollte auch bei der Lagerung die Taupunkttemperatur nicht unterschritten werden. Vorzugsweise findet die Lagerung bei einer relativen Luftfeuchte von unter 65% statt.

Idealerweise erfolgen die Lagerung sowie der Eintrag der mechanischen Energie in ein und derselben Verpackungseinheit, beispielsweise in einer Kiste (K) wie nachfolgenden beschrieben, einem Fass, Sack, Beutel oder ähnlichen üblichen Gebinden.

In einer Ausführungsform der Erfindung werden beide Schritte des erfindungsgemäßen Verfahrens in einer Verpackungseinheit durchgeführt.

Der Eintrag von mechanischer Energie kann auf vielfältige Weise erfolgen. Entscheidend ist, dass den Mentholpartikel mindestens so viel Energie zugeführt wird, wie sie erhalten, wenn man
- 20 kg dieser Mentholpartikel
- in einen Beutel (B) aus einer 0,12 mm dicken Polyethylen-Folie mit den Maßen Länge L(B) von 660 mm und Breite B(B) von 690 mm füllt, den Beutel verschließt,
- diesen Beutel in eine quaderförmige Kiste (K) aus zweiwelliger Wellpappe mit den Innenmaßen Länge L(K) von 385 mm, Breite B(K) von 320 mm und Höhe H(K) von 450 mm und einer Wellpappendicke von 6 mm verpackt, und
- diese Kiste einmalig aus einer Höhe von 1,0 m planparallel mit der von L(K) und H(K) gebildeten Seite auf eine unelastische Oberfläche fallen lässt.

Die Menge der einzutragenden mechanischen Energie kann durch den einmaligen Eintrag der Energie oder durch den mehrmaligen Eintrag von Teilmengen der Energie erfolgen.

Beispiele für geeignete Formen des Eintrags von mechanischer Energie sind horizontales Aufprallen gegen eine Wand; Eintrag von Energie über ein Gewicht oder Stempel auf die ruhenden Mentholpartikel, vorzugsweise als stoßartigen Eintrag; aktiver beschleunigter vertikaler Fall oder der freie Fall. In einer bevorzugten Ausführungsform des vorliegenden Verfahren erfolgt der Eintrag der mechanischen Energie durch den freien Fall auf eine unelastische Oberfläche. Die Fallhöhe kann beispielsweise im Bereich von 1,0 bis 5 m, beispielsweise im Bereich von 1 bis 3 m, beispielsweise bei 1,5 m oder 2 m liegen.

In einer Ausführungsform der vorliegenden Erfindung kann die mechanische Energie zugeführt werden, indem man 20 kg Mentholpartikel
- in einen Beutel (B) aus einer 0,12 mm dicken Polyethylen-Folie mit den Maßen Länge L(B) von 660 mm und Breite B(B) von 690 mm füllt, den Beutel verschließt,
- den Beutel in eine quaderförmige Kiste (K) aus zweiwelliger Wellpappe mit den Innenmaßen Länge L(K) von 385 mm, Breite B(K) von 320 mm und Höhe H(K) von 450 mm und einer Wellpappendicke von 6 mm verpackt, und
- diese Kiste einmalig aus einer Höhe von mindestens 1,0 m bis zu einer Höhe von 5 m, vorzugsweise bis zu einer Höhe von 3 m planparallel mit der von L(K) und H(K) gebildeten Seite auf eine unelastische Oberfläche fallen lässt.

Überraschenderweise wurde gefunden, dass durch die Kombination aus erfindungsgemäßer Lagerung und erfindungsgemäßem Eintrag von mechanischer Energie vorteilhafte, gegen Verbackung stabilisierte Mentholpartikeln erhältlich sind.

Mit dem erfindungsgemäßen Verfahren ist es möglich gegen Verbackung stabilisierte Mentholpartikel zu erhalten, bei denen Form, Größe oder Anzahl der Partikel weitgehend denen der eingesetzten Mentholpartikel entsprechen.

In einer Ausführungsform des Verfahrens wird dieses so geführt, dass die Anzahl der Partikel am Ende des Verfahrens noch mindestens 50%, insbesondere mindestens 60%, vorzugsweise mindestens 70%, vorzugsweise mindestens 80% der zu Beginn des Verfahrens eingesetzten Anzahl an Partikeln beträgt.

In einer Ausführungsform des Verfahren wird dieses so geführt, dass mindestens 50 Gew.-%, insbesondere mindestens 60 Gew.-%, vorzugsweise mindestens 70 Gew.-%, vorzugsweise mindestens 80 Gew.-% der erfindungsgemäßen gegen Verbackung stabilisierten Mentholpartikel dieselbe Form aufweisen wie die eingesetzten Mentholpartikel.

In einer Ausführungsform des Verfahren wird dieses so geführt, dass mindestens 50 Gew.-%, insbesondere mindestens 60 Gew.-%, vorzugsweise mindestens 70 Gew.-%, vorzugsweise mindestens 80 Gew.-% der erfindungsgemäßen gegen Verbackung stabilisierten Mentholpartikel dieselbe Größe aufweisen wie die eingesetzten Mentholpartikel.

Der Fachmann kann diese Ausführungsformen beispielsweise durch Begrenzung des Eintrags der mechanischen Energie oder die Wahl der Art des Eintrags der mechanischen Energie ausführen.

In einer Ausführungsform der Erfindung wird das Verfahren so geführt, dass man den Mentholpartikeln nur so viel Energie zuführt, dass mindestens 50 Gew.-%, vorzugsweise mindestens 60 Gew.-%, insbesondere mindestens 70 Gew.-%, vorzugsweise mindestens 80 Gew.-% der Partikel ihre Größe behalten.

In einer Ausführungsform der Erfindung wird das Verfahren so geführt, dass man den Mentholpartikeln nur so viel Energie zuführt, dass mindestens 50 Gew.-%, vorzugsweise mindestens 60 Gew.-%, insbesondere mindestens 70 Gew.-%, vorzugsweise mindestens 80 Gew.-% der Partikel ihre Form behalten.

Als Mentholpartikel im Sinne der Erfindung werden diskrete Teilchen von Menthol in fester Form verstanden.

Die Begriffe "Mentholpartikel", "einzusetzende Mentholpartikel" sowie "eingesetzte Mentholpartikel" bezeichnet die als Ausgangsprodukte für das erfindungsgemäße Verfahren einzusetzenden Partikel. Der Begriff "gegen Verbackung stabilisierte Mentholpartikel" bezeichnet die nach dem erfindungsgemäßen Verfahren erhältlichen Partikel.

Die Größe der Mentholpartikel bezeichnet die längste Ausdehnung im Raum eines Partikels einer beliebigen Form. So ist bei Mentholpartikeln in Kugelform die Größe des Partikel der Durchmesser der Kugel, bei Mentholpartikeln in Quaderform ist die Größe des Partikels die Raumdiagonale des Quaders.

In einer bevorzugten Ausführungsform der Erfindung werden Mentholpartikel eingesetzt, die eine Größe im Bereich von 1 bis 35 mm, insbesondere von 4 bis 35 mm, insbesondere von 5 bis 30 mm, vorzugsweise von 10 bis 25, vorzugsweise von 12 bis 24, insbesondere 15 bis 20 mm aufweisen.

In einer bevorzugten Ausführungsform der Erfindung werden Mentholpartikel eingesetzt, die eine Größe im Bereich von 4 bis 35 mm, insbesondere im Bereich von 5 bis 30 mm, vorzugsweise von 10 bis 25, vorzugsweise im Bereich von 12 bis 24, insbesondere 15 bis 20 mm aufweisen und deren Anteil an Mentholpartikeln mit einer Größe von kleiner 4 mm weniger als 5 Gew.-%, vorzugsweise als 2 Gew.-% und insbesondere weniger als 1 Gew.-%, ganz bevorzugt weniger als 0,5 Gew.-%, besonders bevorzugt weniger als 0,1 Gew.-% beträgt.

Die Größe der Partikel sowie die Partikelgrößenverteilung kann beispielsweise mikroskopisch oder durch Siebanalyse bestimmt werden.

Als Formkörpern wird die Geometrie einer dreidimensionalen Figur bezeichnet, die durch ihre Oberflächen beschrieben wird. Die Oberfläche eines Formkörpers kann dabei aus flachen oder gewölbten Flächen zusammengesetzt sein. Der Begriff Form wird im Folgenden synonym zum Begriff Formkörper verwendet.

Mentholpartikel sind in unterschiedlichsten Formkörper bekannt, wie z.B. Kristallnadeln, Polyeder, Würfel, Quader; Kegel, Prisma, Kugeln, Halbkugeln, Kugelscheiben, Zylinder, Tropfen, sowie Mischformen daraus etc. Die Formkörper ergeben sich dabei aus der Art der Herstellung:
Kristallnadeln werden z.B. durch Kristallisation von L-Menthol aus einer L-Menthol-haltigen Lösung oder Schmelze gewonnen. Kristallnadeln weisen üblicherweise eine Länge von 0,5 bis 3 cm und eine Dicke von 1 bis 3 mm auf. Kristallnadeln sind aufgrund ihres Herstellungsverfahrens durch eine Geometrie des Formkörpers gekennzeichnet, bei der nur eine Ausdehnung im Raum ein Vielfaches der übrigen Ausdehnungen im Raum ausmacht. Presslinge (synonym kompaktierte Kristalle/ Tabletten) werden durch Verpressung von Kristallen erhalten und können je nach Pressform beispielsweise als Kugel, Würfel, Quader, Kissen oder Stränge vorliegen. Pastillen können z.B. durch Vertropfung von Mentholschmelzen auf eine gekühlte Oberfläche erhalten werden. Sphärische Partikel können z.B. durch Vertropfung von Mentholschmelzen in Wasser erhalten werden. Schuppen können z.B. durch Inkontaktbringen einer Mentholschmelze mit zwei voneinander beabstandeten gekühlten Flächen erhalten werden.

Das erfindungsgemäße Verfahren eignet sich für Mentholpartikel unabhängig von ihrem Formkörper. Bevorzugte Mentholpartikel im Sinne der Erfindung sind Mentholpartikel in Form von Schuppen, Sphären oder Pastillen. Besonders bevorzugt sind Mentholpartikel in Form von Schuppen.

Schuppen im Sinne der vorliegenden Erfindung sind Formkörper, bei denen mindestens 2 Flächen der Formkörper zueinander parallel sind. Als Schuppen im Sinne der vorliegenden Erfindung werden insbesondere solche Formkörper verstanden, bei denen die 2 größten Flächen der Formkörper zueinander parallel sind.

Der Abstand der 2 zueinander parallelen Flächen wird als Dicke der Schuppe bezeichnet. Sofern mehr als 2 Flächen zueinander parallel sind, wird der Abstand der 2 Flächen, welcher der kleinste ist, als Dicke der Schuppe bezeichnet.

Als Schuppen sind insbesondere solche Formköper zu verstehen, bei denen mindestens 2 Flächen zueinander parallel sind und bei denen die weiteren Ausdehnungen des Formkörpers im Raum (als mittlere Kantenlänge der Schuppe bezeichnet) mindestens das 1,25fache der Dicke betragen.

Als Schuppen sind insbesondere solche Formköper zu verstehen, bei denen mindestens 2 Flächen zueinander parallel sind, ausgenommen solche Formkörper, bei denen nur eine Ausdehnung des Formkörpers im Raum ein Vielfaches (mindestens 3faches) der Dicke beträgt.

Beispiele für Schuppen sind somit Quader oder Würfel. Schuppen sind weiterhin solche Formkörper, die durch Zerkleinerung, insbesondere Brechung, eines Films erzeugt wurden und bei denen die nicht von den parallelen Flächen gebildeten Flächen nicht im rechten Winkel zu den von den parallelen Flächen gebildeten Flächen ausgebildet sind.

Sphären im Sinne der vorliegenden Erfindung sind Formkörper, bei denen alle Flächen des Formkörpers gewölbt sind. Beispiele für Sphären sind demnach Kugeln, Kissen oder Tropfen.

Pastillen im Sinne der vorliegenden Erfindung sind Formen, welche durch eine planare und eine der planaren Fläche gegenüberliegende, gewölbte Fläche gekennzeichnet sind.

Mentholpartikel, welche als Ausgangsprodukte für das erfindungsgemäße Verfahren geeignet sind, sind beispielsweise die in WO2008/152009 (BASF) oder US 3,023,253 und US 3,064,311 beschriebenen Schuppen, Pastillen gemäß WO 2016/034481 (Sandvik), Pastillen gemäß WO2016/016154 (Symrise) oder Presslinge nach WO 03/101924 (Symrise).

Besonders bevorzugt sind Mentholpartikel, in denen das Menthol als L-Menthol vorliegt. Besonders bevorzugt sind Mentholpartikel in denen mehr als 80 Gew.-%, insbesondere mehr als 90, insbesondere mehr als 99,5, vorzugsweise mehr als 99,7 Gew.-%, Menthol, insbesondere L-Menthol, bezogen auf das Gesamtgewicht des Partikels vorliegt.

Als Ausgangsstoff zur Herstellung der erfindungsgemäß einzusetzenden Mentholpartikel eignen sich Schmelzen von L-Menthol der Formel (I), 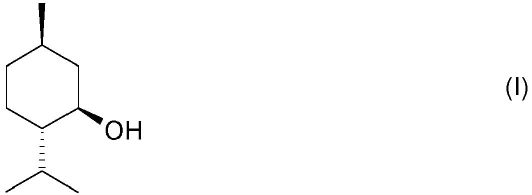 IUPAC Name: 1R, 2S, 5R 2-isopropyl-5-methylcyclohexanol
wobei das geschmolzene Menthol natürlichen oder synthetischen Ursprungs sein kann und einen Enantiomerenüberschuss von üblicherweise mindestens 95, 96 oder 97% ee bis 100% ee, bevorzugt 98, 98,5 oder 99 bis 99,9 % ee aufweist. Insbesondere eignen sich als Ausgangsstoffe im Rahmen des erfindungsgemäßen Verfahrens solche Schmelzen von L-Menthol, die einen Gehalt an L-Menthol von mindestens 95, 96 oder 97 Gew.-% oder darüber, bevorzugt mindestens 98 bis 100 Gew.-% und ganz besonders bevorzugt 98, 98,5 oder 99 bis 99,9 Gew.-% aufweisen (jeweils bezogen auf das Gesamtgewicht der Schmelze), neben Verunreinigungen wie beispielsweise Resten von Lösemitteln, Diastereomeren des L-Menthols der Formel (I) oder Nebenprodukten aus Synthese- bzw. Isolierverfahren.

Bevorzugt wird eine L-Menthol-Schmelze eingesetzt, die 0,1 bis 50 Gew.-%, insbesondere 1 bis 40 Gew.-%, insbesondere 5 bis 35 Gew.-%, vorzugsweise 10 bis 30 Gew.-% Impfkristalle von Menthol enthält.

Die Angabe der Gew.-% an Imfpkristallen ist jeweils bezogen auf das Gesamtgewicht des einzusetzenden Gemisches aus Schmelze und Impfkristallen.

Bevorzugt wird eine L-Menthol-Schmelze eingesetzt, die 0,1 bis 50 Gew.-%, insbesondere 1 bis 40 Gew.-%, insbesondere 5 bis 35 Gew.-%, vorzugsweise 10 bis 30 Gew.-% Impfkristalle von Menthol enthält, wobei die restlichen Gewichtanteile die Menge an L-Menthol in geschmolzener Form ausmachen.

Als L-Menthol-Schmelze ist somit beispielsweise solches L-Menthol zu verstehen, welches 50 bis 99,9 Gew.-%, 60 bis 99 Gew.-%, insbesondere 65 bis 95 Gew.-% vorzugsweise 70 bis 90 Gew.-% Menthol in geschmolzener Form enthält.

Bei der bevorzugt einzusetzenden L-Menthol-Schmelze handelt es sich demnach um eine Suspension aus Impfkristallen und geschmolzenem L-Menthol.

Solche als Impfkristalle zu bezeichnenden Kristalle von L-Menthol können beispielsweise auf herkömmliche Weise durch Kristallisation von L-Menthol aus einer L-Menthol-haltigen Lösung oder Schmelze gewonnen werden und der L-Menthol-Schmelze zugesetzt werden. Dies kann beispielsweise durch Einrühren in ein Vorlagegefäß oder Aufstreuen von zuvor zerkleinerten Kristallen des L-Menthols auf die eingesetzte L-Menthol-Schmelze (den flüssigen Kristallfilm) erfolgen. Es ist auch möglich die Impfkristalle durch Einsatz eines Kratzkühler oder Extruders wie nachfolgend beschrieben zu erzeugen und der L-Menthol-Schmelze zu zusetzen. Es ist weiterhin möglich als Impfkristalle den unten beschriebenen Feinanteil zu verwenden.

Es ist auch möglich die Impfkristalle in der L-Menthol-Schmelze vor dem Inkontaktbringen mit der gekühlten Oberfläche zu erzeugen. Geeignete Verfahren zur Erzeugung der Impfkristalle in der L-Menthol-Schmelze sind beispielsweise der Einsatz eines Kratzkühlers oder der Einsatz eines Extruders.

Eine Ausführungsform der Erfindung zeichnet sich dadurch aus, dass die Impfkristalle durch Behandlung der einzusetzenden Menthol-Schmelze in einem Kratzkühler gebildet werden.

In einer bevorzugten Ausführungsform wird die Impfung durch Passage der Schmelze durch einen unterhalb des Schmelzpunktes betriebenen Wärmetauscher erreicht, dessen Wände durch ein Abriebsorgan von aufkristallisiertem Material befreit werden, wobei das Abriebsorgan aus mindestens einem, vorzugsweise mehreren, flächenförmigen Elementen besteht. Beispiele für flächenförmige Elemente sind Schaber oder Kratzer. Solche Anordnungen sind als Schabeoder Kratzkühler bekannt und beispielweise in G. Arkenbout, Melt Crystallization Technology, Technomic Publishing Co. 1995, S. 230 beschrieben. Geeignete Kratzkühler weisen beispielsweise eine kreiszylindrische, gekühlte Innenfläche auf, die von der auf einer rotierenden Welle angeordneten Kratzern überstrichen wird.

Im Rahmen einer Ausführungsform setzt man solche Impfkristalle von L-Menthol ein, die durch Behandlung der einzusetzenden L-Menthol Schmelze in einem Kratzkühler gewonnen werden, wobei die Impfkristalle in situ in der zu verfestigenden L-Menthol Schmelze gebildet werden, wodurch ein zusätzlicher Arbeitsschritt vermieden wird.

In einer Ausführungsform wird die Temperatur im Kratzkühler auf einen Bereich von 10 bis 32 °C, insbesondere von 15 bis 20 °C eingestellt. Die Temperatureinstellung kann mittels flüssigem Kühlmittel, insbesondere Wasser erfolgen. Die Menthol-Schmelze kann in einem einmaligen Durchlauf durch den Kratzkühler befördert werden. Ebenso ist es möglich, die MentholSchmelze im Kreislauf mehrfach durch den Kratzkühler zu befördern, so lange bis der gewünschte Anteil an Impfkristallen erzeugt wurde.

Eine Ausführungsform zeichnet sich dadurch aus, dass die Impfkristalle durch Behandlung der einzusetzenden Menthol-Schmelze in einem Extruder gebildet werden.

So lassen sich Menthol-Schmelzen, insbesondere L-Menthol-Schmelzen, welche 0,1 bis 50 Gew.-% an Impfkristallen von Menthol, insbesondere L-Menthol, enthalten, dadurch erhalten, dass man eine Menthol-Schmelze durch einen Extruder führt.

Ein Wärmetauscher mit einem Abriebsorgan, welches mindestens ein schneckenförmiges Element enthält, wird im Sinne der Erfindung als Extruder bezeichnet. Solche Anordnungen sind beispielweise in C.M. Van't Land, Industrial Crystallization of Melts, Marcel Dekker 2005, S.161 - 167 beschrieben.

Das Abriebsorgan kann eine oder mehrere Vorrichtungen mit schneckenförmigen Elementen umfassen, so z.B. eine Schnecke oder insbesondere zwei Schnecken (sog. Doppelschnecke). Die Vorrichtungen können gleich- oder gegenläufig angeordnet sein. Die Vorrichtungen können ineinandergreifend oder nicht ineinandergreifend angeordnet sein. Neben den schneckenförmigen Elementen, die in der Regel hauptsächlich der Förderung dienen, kann jede Vorrichtung des Abriebsorgans weitere Elemente enthalten, die üblicherweise hauptsächlich der Durchmischung dienen. Als Beispiele für solche Elemente seien Knetblöcke genannt. Üblicherweise weist die Vorrichtung des Abriebsorgans verschiedene Zonen von schneckenförmigen Elementen und Durchmischungselementen auf.

In einer Ausführungsform wird als Extruder ein Wärmetauscher mit einer gegenläufigen Doppelschnecke eingesetzt.

In einer Ausführungsform wird als Extruder ein Wärmetauscher mit einer gleichläufigen Doppelschnecke eingesetzt. Vorzugsweise wird als Extruder ein Wärmetauscher mit einer gleichläufigen Doppelschnecke eingesetzt, bei der die Schnecken ineinandergreifend angeordnet sind.

Die Menthol-Schmelze kann durch die Rotation des Abriebsorgans durch den Extruder befördert werden oder zusätzlich, beispielsweise durch eine Pumpe, unterstützt durch den Extruder befördert werden.

In einer Ausführungsform wird die Temperatur des Extruders auf unter 42°C eingestellt, vorzugsweise auf eine Temperatur von 5 bis 40 °C, insbesondere auf einen Bereich von 10 bis 32°C, insbesondere von 15 bis 20 °C. In einer weiteren Ausführungsform kann die Temperatur des Extruders am Einlass und/oder am Auslass auf höhere Temperaturen als im dazwischen liegenden Bereich eingestellt werden. Die Temperatureinstellung kann mittels flüssigem Kühlmittel, insbesondere Wasser erfolgen. Die Menthol-Schmelze kann in einem einmaligen Durchlauf durch den Extruder befördert werden. Ebenso ist es möglich, die Menthol-Schmelze im Kreislauf mehrfach durch den Extruder zu befördern, so lange bis der gewünschte Anteil an Impfkristallen erzeugt wurde.

Im Rahmen einer Ausführungsform setzt man solche Impfkristalle von L-Menthol ein, die durch Behandlung der erfindungsgemäß einzusetzenden L-Menthol Schmelze in einem Extruder gewonnen werden, wobei die Impfkristalle in situ in der zu verfestigenden L-Menthol Schmelze gebildet werden, wodurch ein zusätzlicher Arbeitsschritt vermieden wird.

Der Anteil an Impfkristallen in der Menthol-Schmelze kann beispielsweise über die Messung der Dichte, die Messung Viskosität der L-Menthol-Schmelze, die Leistungsaufnahme des Kratzkühlers oder optisch, beispielsweise mit Hilfe einer Streulichtsonde bestimmt werden.

In einer bevorzugten Ausführungsform wird der Anteil an Impfkristallen in der L-MentholSchmelze über die Messung der Dichte bestimmt. Die Dichte von L-Menthol Kristallen in der alpha-Modifikation liegt bei 900 kg/m³ bei 15 °C. Die Dichte von Menthol-Schmelzen bei verschiedenen Temperaturen ist beispielsweise in Ishchenki, E.D.; Roshchina, G.P. Ukr Fiz Zh Ukr. Ed., 1963, Vol 8, Issue 11 Seiten 1241-1249 beschrieben.

Die Messung des Anteils an Impfkristallen erfolgt üblicherweise am Auslass des Kratzkühler bzw. des Extruders. Sofern der gewünschte Anteil an Impfkristallen nicht erreicht ist, kann die Menthol-Schmelze erneut dem Kratzkühler bzw. Extruder zugeführt werden, solange bis der gewünschte Anteil an Impfkristallen in der Menthol-Schmelze erreicht ist. Alternativ können der L-Menthol-Schmelze Impfkristalle zugesetzt werden.

Die für die Herstellung der Mentholpartikel einzusetzende L-Menthol-Schmelze wird üblicherweise bei einer Temperatur im Bereich von etwa 40 bis 60°C, bevorzugt etwa 43 bis 50°C eingesetzt. Dabei handelt es sich bei L-Menthol-Schmelzen im Temperaturbereich von unterhalb von 42 bis 43°C, d.h. unterhalb des Schmelzpunktes von L-Menthol, um unterkühlte Schmelzen.

In einer bevorzugten Ausführungsform des erfindungsgemäßen Verfahrens setzt man Mentholpartikel in Form von Schuppen ein. Diese können beispielsweise durch Aufbringen einer Schmelze von L-Menthol auf einer gekühlten Oberfläche, wie beispielsweise einer Tauchwalze, erhalten werden. Der auf der Tauchwalze ankristallisierte Mentholfilm kann nachbehandelt werden, indem er durch Wärmeeintrag getempert und durch Aufbringen zusätzlichen Menthols verstärkt wird. Mentholpartikel in Form von Schuppen können weiterhin beispielsweise erhalten werden durch das Aufbringen einer Mentholschmelze zwischen ein Paar gegenläufig rotierender, gekühlter Walzen.

In einer bevorzugten Ausführungsform des Verfahren setzt man Mentholpartikel in Form von Schuppen mit einer Dicke von 0,2 bis 15, bevorzugt von 4 bis 10 mm, insbesondere 6 bis 8 mm ein.

In einer bevorzugten Ausführungsform des Verfahren setzt man Mentholpartikel in Form von Schuppen mit einer mittleren Kantenlägen von 5 bis 25 mm, vorzugsweise 12 bis 16 mm ein.

In einer bevorzugten Ausführungsform des Verfahren setzt man Mentholpartikel in Form von Schuppen mit einer Dicke von 0,2 bis 15, bevorzugt von 4 bis 10 mm, insbesondere 6 bis 8 mm und einer mittleren Kantenlägen von 5 bis 25 mm, vorzugsweise 12 bis 16 mm ein.

In einer bevorzugten Ausführungsform des Verfahrens setzt man Mentholpartikel in Form von Schuppen ein, die eine Größe im Bereich von 1 bis 35 mm, insbesondere von 4 bis 35 mm, insbesondere von 5 bis 30 mm, vorzugsweise von 10 bis 25, vorzugsweise von 12 bis 24, insbesondere 15 bis 20 mm aufweisen.

In einer bevorzugten Ausführungsform des Verfahrens setzt man Mentholpartikel in Form von Schuppen ein, die eine Größe im Bereich von 4 bis 35 mm, insbesondere im Bereich von 5 bis 30 mm, vorzugsweise von 10 bis 25, vorzugsweise im Bereich von 12 bis 24, insbesondere 15 bis 20 mm aufweisen und deren Anteil an Mentholpartikeln mit einer Größe von kleiner 4 mm weniger als 5 Gew.-%, vorzugsweise als 2 Gew.-% und insbesondere weniger als 1 Gew.-%, ganz bevorzugt weniger als 0,5 Gew.-%, besonders bevorzugt weniger als 0,1 Gew.-% beträgt.

In einer bevorzugten Ausführungsform des Verfahren setzt man Mentholpartikel in Form von Schuppen ein, die erhalten wurden durch Inkontaktbringen einer Menthol-Schmelze mit zwei voneinander beabstandeten gekühlten Oberflächen unter Erstarrung der Menthol-Schmelze zu Menthol, wobei man den Kontakt zwischen der erstarrenden Menthol-Schmelze und den gekühlten Oberflächen mindestens bis zum Abschluss der Erstarrung aufrechterhält.

Vorteilhafterweise werden die oben beschriebenen Menthol-Schmelzen eingesetzt. Bevorzugt wird eine L-Menthol-Schmelze eingesetzt, die 0,1 bis 50 Gew.-%, insbesondere 1 bis 40 Gew.%, insbesondere 5 bis 35 Gew.-%, vorzugsweise 10 bis 30 Gew.-% Impfkristalle von Menthol enthält.

Die L-Menthol-Schmelze kann beispielsweise über ein Wehr oder über eine temperierte Rohrleitung zu den zwei voneinander beabstandeten, gekühlten Oberflächen transportiert werden. Die eingesetzte L-Menthol-Schmelze wird in dieser Ausführungsform mit zwei voneinander beabstandeten, gekühlten Oberflächen in Kontakt gebracht. Bevorzugt befindet sich die eingesetzte L-Menthol-Schmelze im Zwischenraum zwischen den beiden beabstandeten, gekühlten Oberflächen. Das Inkontaktbringen der Schmelze mit den einzelnen Oberflächen kann simultan, d.h. gleichzeitig erfolgen oder zeitlich versetzt erfolgen.

Üblicherweise, bedingt durch verfahrenstechnische Erfordernisse, erfolgt das Inkontaktbringen der eingesetzten L-Menthol-Schmelze mit den beiden gekühlten Oberflächen zeitlich versetzt, zum Beispiel indem die Schmelze zunächst auf einen nicht aktiv gekühlten Bereich einer der beiden Oberflächen aufgegeben wird, diese dann kurze Zeit darauf gekühlt wird und zusätzlich mit der zweiten gekühlten Oberfläche in Kontakt kommt.

Üblicherweise, bedingt durch verfahrenstechnische Erfordernisse, erfolgt das Inkontaktbringen der eingesetzten L-Menthol-Schmelze mit den beiden gekühlten Oberflächen zeitlich versetzt, so dass die Schmelze zunächst mit einer und kurze Zeit darauf zusätzlich mit der zweiten gekühlten Oberfläche in Kontakt kommt. Es hat sich dabei als vorteilhaft erwiesen, die Zeitspanne zwischen dem Inkontaktbringen der L-Menthol-Schmelze mit den jeweiligen gekühlten Oberflächen möglichst gering zu halten, so dass, je nach Temperaturdifferenz zwischen eingesetzter L-Menthol-Schmelze und als erstes in Kontakt gebrachter gekühlter Oberfläche, noch keine weitgehende oder vollständige Verfestigung der eingesetzten L-Menthol Schmelze erfolgt ist, bevor der Kontakt mit der zweiten gekühlten Oberfläche hergestellt ist. Üblicherweise beträgt die Zeitspanne zwischen dem Inkontaktbringen der eingesetzten L-Menthol-Schmelze mit der jeweiligen Oberfläche nicht mehr als 30 s, bevorzugt bis zu 20 s und besonders bevorzugt bis zu 10 s.

Im Rahmen einer Ausführungsform handelt es sich bei den einzusetzenden gekühlten Oberflächen jeweils um glatte Oberflächen, bevorzugt um ebene Teilbereiche endloser, aus Stahl, anderen Metallen, Kunststoffen oder Kombinationen dieser Materialien gefertigter Bänder. Besonders bevorzugt sind endlose Bänder aus glattem oder poliertem Edelstahl.

Auch die Dauer des Kontakts der eingesetzten Schmelze, bzw. der erstarrenden Schmelze mit den beiden gekühlten Oberflächen, im Folgenden als Kontaktzeit bezeichnet, kann für die einzelnen Oberflächen gleich oder unterschiedlich lang sein. Üblicherweise ist die Kontaktzeit der Schmelze mit den jeweiligen gekühlten Oberflächen verschieden lang, da, wie vorstehend dargestellt, das Inkontaktbringen oft zeitlich versetzt erfolgt und in der Regel auch das Ende der Kontaktzeit, d.h. das Beenden des Kontakts der vollständig erstarrten L-Menthol-Schmelze mit der jeweiligen gekühlten Oberfläche zu verschiedenen Zeitpunkten erfolgt. Unabhängig von der zeitlichen Abfolge des Inkontaktbringens der Schmelze mit den einzelnen der beiden gekühlten Oberflächen und dem Ablösen der vollständig erstarrten L-Menthol-Schmelze von den Oberflächen überlappen die Kontaktzeiten bezüglich der einzelnen gekühlten Oberflächen zeitlich, so dass die eingesetzte L-Menthol-Schmelze bzw. die erstarrende L-Menthol-Schmelze über einen wählbaren Zeitraum gleichzeitig mit beiden gekühlten Oberflächen in Kontakt ist.

In dieser Ausführungsform erhält man den Kontakt zwischen der erstarrenden L-MentholSchmelze und den gekühlten Oberflächen mindestens bis zum Abschluss der Erstarrung aufrecht. Die Erstarrung bzw. Kristallisation der eingesetzten L-Menthol-Schmelze wird vorzugsweise erst dann als abgeschlossen betrachtet, wenn das erhaltene L-Menthol in fester Form zu mindestens etwa 80 Gew.-% oder besser 85 bis 100 Gew.-%, bevorzugt zu 90 bis 100 Gew.-%, bevorzugt zu 95 oder 97 bis 99,5 Gew.-% und ganz besonders bevorzugt zu 98 bis 99 Gew.-% in der alpha-Modifikation vorliegt. Die jeweils vorliegende Modifikation des erhaltenen verfestigten L-Menthols kann mit dem Fachmann bekannten Verfahren wie Röntgenbeugung oder Pulverdiffraktometrie ermittelt werden (s. z.B. Joel Bernstein, Polymorphism in Molecular Crystals, Oxford University Press 2002, S. 94-150).

Unter dem Begriff "gekühlte Oberflächen" sind solche Oberflächen zu verstehen, die eine Temperatur unterhalb des Schmelz- bzw. Erstarrungspunktes von L-Menthol von 42 bis 43°C aufweisen bzw. auf eine solche Temperatur temperiert sind. Die einzusetzenden gekühlten Oberflächen weisen unabhängig voneinander üblicherweise jeweils eine Temperatur im Bereich von etwa 0 bis etwa 40°C, bevorzugt von etwa 0 bis etwa 35°C, besonders bevorzugt 5 bis 30°C und ganz besonders bevorzugt im Bereich von 10 bis 25°C, insbesondere von 15 bis 20°C auf. Dabei können beide Oberflächen die gleiche oder eine unterschiedliche Temperatur aufweisen. Es ist auch möglich, die Temperatur der gekühlten Oberflächen individuell, im Verlauf der jeweiligen Kontaktzeit zu verändern, d.h. anzuheben oder abzusenken.

Im Rahmen einer bevorzugten Ausführungsform weisen die beiden gekühlten Oberflächen eine planparallele Ausrichtung mit einem Abstand von üblicherweise von 0,2 bis 15, vorzugsweise 2 bis 10, insbesondere 3 bis 9, insbesondere 5 bis 8 mm insbesondere 6 bis 8 mm zueinander auf.

Unter dem Begriff planparallele Ausrichtung ist dabei zu verstehen, dass die beiden gekühlten Oberflächen im Rahmen üblicher Messgenauigkeiten über den gesamten, mit der zu verfestigenden L-Menthol Schmelze in Kontakt gebrachten Bereich oder Teilbereich den gleichen Abstand aufweisen. Der zwischen den beiden gekühlten Oberflächen gebildete Zwischenraum ist vorteilhafterweise vollständig mit L-Menthol angefüllt, weil so eine möglichst große Kontaktfläche zwischen den gekühlten Oberflächen und der zu verfestigenden L-Menthol-Schmelze gegeben ist.

In Abhängigkeit vom Anteil an Impfkristallen der eingesetzten L-Menthol-Schmelze, von den gewählten Temperaturen der eingesetzten L-Menthol-Schmelze, sowie dem Abstand und den Temperaturen der beiden gekühlten Oberflächen wird die Kontaktzeit der erstarrenden L-Menthol-Schmelze mit den beiden gekühlten Oberflächen so gewählt, dass die Erstarrung abgeschlossen ist. Übliche Kontaktzeiten liegen im Bereich von 10 bis 300 s, vorzugsweise 120 bis 240 s. Üblicherweise ist die Erstarrung nach einer Kontaktzeit von etwa 10 bis etwa 300 s, bevorzugt etwa 20 bis etwa 250 s, bevorzugt bis etwa 200 s und ganz besonders bevorzugt von 30 bis 150 s, bevorzugt bis 100 s abgeschlossen. Dabei sind die genannten Kontaktzeiten so zu verstehen, dass sie die Zeitspannen angeben, während derer gleichzeitiger Kontakt zwischen der L-Menthol-Schmelze bzw. der erstarrenden oder bereits erstarrten L-Menthol-Schmelze zu beiden gekühlten Oberflächen besteht. Es ist auch möglich, den Kontakt der erstarrten L-Menthol-Schmelze mit einer der beiden gekühlten Oberflächen darüber hinaus auszudehnen.

Kurze Kontaktzeiten und eine vollständige Erstarrung können im Rahmen einer bevorzugten Ausführungsform erreicht werden, in dem die Schmelze vor oder während des Inkontaktbringens mit den gekühlten Oberflächen wie vorstehend beschrieben mit Impfkristallen versetzt wird.

Im Rahmen einer besonders bevorzugten Ausführungsform führt man das Verfahren unter Einsatz eines Doppelbandkühlers durch. Doppelbandkühler sind dem Fachmann bekannt und können beispielsweise von den Firmen Ipco Germany GmbH, 70736 Fellbach, Deutschland oder SBS Steel Belt Systems S.r.L., Italien bezogen werden.

Bei Einsatz der genannten Doppelbandkühler werden die einzusetzenden gekühlten Oberflächen in Form zweier in entgegengesetztem Drehsinn über Walzen geführter, üblicherweise aus Stahl gefertigter Endlosbänder (Kühlbändern) realisiert (s. C.M. van't Land, Industrial Crystallization of Melts, Marcel Dekker 2005, S. 63). Die Erstarrung der L-Menthol-Schmelze zu L-Menthol in fester Form findet dann im Zwischenraum zwischen den planparallel verlaufenden, einander zugewandten Teilbereichen der beiden Kühlbänder des Doppelbandkühlers statt.

Um ein möglichst gleichzeitiges Inkontaktbringen der zu verfestigenden L-Menthol-Schmelze mit den beiden Kühlbändern zu erreichen, empfiehlt es sich, die Schmelze möglichst nah an der Stelle mit den Kühlbändern in Kontakt zu bringen, an der der Zwischenraum zwischen den planparallel verlaufenden Teilbereichen der beiden Kühlbänder beginnt, so dass es in möglichst geringem Ausmaß zu einer vorzeitigen Verfestigung der L-Menthol-Schmelze kommt.

Das bevorzugte Verfahren zur Herstellung von Mentholpartikel in Form von Schuppen kann diskontinuierlich, beispielsweise unter Einsatz gekühlter Stempel oder, beispielsweise unter Einsatz eines wie vorstehend genannten Doppelbandkühlers, kontinuierlich durchgeführt werden. Dabei eröffnet insbesondere die kontinuierliche Verfahrensweise wirtschaftliche Vorteile.

Der erhaltene, erstarrte L-Mentholfilm kann dann nach dem Fachmann bekannten Verfahren von der bzw. den gekühlten Oberflächen entfernt werden. Je nach Verfahrensführung fällt der L-Mentholfilm direkt von der gekühlten Oberfläche ab oder er kann durch Einsatz eines angestellten Messers von einer oder beiden der gekühlten Oberflächen abgelöst werden. Löst man den erstarrten L-Mentholfilm von einer oder beiden der gekühlten Oberflächen, vorzugsweise den Kühlbändern eines bevorzugt einzusetzenden Doppelbandkühlers ab, erhält man L-Mentholpartikel in Form von Schuppen.

Diese können mit geeigneten Nachbehandlungsmethoden auf L-Menthol-Schuppen der gewünschten Größe eingestellt werden. Als Nachbehandlungsmethoden seien beispielsweise die Zerkleinerung, z.B. die Zerkleinerung mittels Stiftbrechern, Daumenbrechern, Einwellenzerkleinerern, Rotorscheren, Schlagbrechern oder mittels Backenbrechern sowie die Siebzerkleinerung genannt. Bevorzugt werden für die Zerkleinerung Mahlwerke mit langsamen Geschwindigkeiten, wie beispielsweise im Bereich von 0,2 m/s bis 10 m/s, insbesondere im Bereich von 0,5 m/s bis 2,0 m/s eingesetzt.

Weiterhin kann eine Abtrennung von Partikeln einer bestimmten Größe durch Siebung erfolgen. Die genannten Nachbehandlungsmethoden können in beliebiger Kombination miteinander eingesetzt werden.

Die so erhaltenen Mentholpartikel in Schuppenform können (vor oder nach einer gegebenenfalls durchgeführten Nachbehandlung) noch durch weitere Kühlung, beispielsweise auf einer gekühlten Förderschnecke oder einem gekühlten Förderband, behandelt werden.

Durch das bevorzugte Verfahren zur Herstellung von Mentholpartikel in Form von Schuppen sind L-Mentholpartikel in Form von Schuppen erhältlich, die, bedingt durch die Erstarrung im Kontakt mit zwei gekühlten Oberflächen, zumindest zwei glatte Oberflächen aufweist.

Die durch das bevorzugte Verfahren zur Herstellung von Mentholpartikel in Form von Schuppen erhältlichen Mentholpartikel weisen, je nach gewähltem Abstand der beiden gekühlten Oberflächen, eine Dicke von 0,2 bis 15, vorzugsweise 2 bis 10, insbesondere 3 bis 9, insbesondere 5 bis 8 mm insbesondere 6 bis 8 mm auf.

Die Größe der Schuppen kann je nach Art der Nachbehandlung frei gewählt werden und reicht von endlosen Bändern bis hin zu stark zerkleinerten Schuppen. In einer Ausführungsform kann die Abtrennung von Partikeln mit einer Größe von unter 4 mm (sogenannter Feinanteil) erfolgen, beispielsweise durch Siebung. In einer Ausführung kann der so abgetrennte Feinanteil dann der zu verfestigenden L-Menthol-Schmelze als Impfkristall zugesetzt werden.

In einer bevorzugten Ausführungsform werden Mentholpartikel in Form von Schuppen erhalten, die eine Größe im Bereich von 1 bis 35 mm, insbesondere von 4 bis 35 mm, insbesondere von 5 bis 30 mm, vorzugsweise von 10 bis 25, vorzugsweise von 12 bis 24, insbesondere 15 bis 20 mm aufweisen.

In einer bevorzugten Ausführungsform werden Mentholpartikel in Form von Schuppen erhalten, die eine Größe im Bereich von 4 bis 35 mm, insbesondere von 5 bis 30 mm, vorzugsweise von 10 bis 25, vorzugsweise von 12 bis 24, insbesondere 15 bis 20 mm aufweisen und deren Anteil an Mentholpartikeln mit einer Größe von kleiner 4 mm weniger als 5 Gew.-%, vorzugsweise als 2 Gew.-% und insbesondere weniger als 1 Gew.-%, ganz bevorzugt weniger als 0,5 Gew.-%, besonders bevorzugt weniger als 0,1 Gew.-% beträgt.

Die so erhältlichen L-Mentholpartikel in Form von Schuppen, eignen sich insbesondere als einzusetzende Mentholpartikel zur Herstellung von gegen Verbackung stabilisierten Mentholpartikeln.

Ein weiterer Gegenstand der Erfindung betrifft die Verwendung der nach dem erfindungsgemäßen Verfahren erhältlichen, gegen Verbackung stabilisierten Mentholpartikel zur Herstellung von oder in Gebrauchs- oder Verbrauchsgüter wie beispielsweise pharmazeutische oder kosmetische Zubereitungen, Nahrungsmittel, Hygiene- oder Reinigungsartikel, Süßwaren oder Tabakerzeugnisse.

Ein weiterer Gegenstand der Erfindung betrifft nach dem erfindungsgemäßen Verfahren erhältliche gegen Verbackung stabilisierte Mentholpartikel.

Ein generelles Problem bei der Lagerung von Mentholpartikel ist ihre eingeschränkte Lagerfähigkeit. Mentholpartikel, insbesondere in Form von Schuppen, Sphären und Pastillen zeigen nach Lagerung sogenannte Verbackungserscheinungen. Diese schränken den Einsatz der gelagerten Partikel erheblich ein.

Eine Aufgabe der vorliegenden Erfindung bestand daher in der Bereitstellung von Mentholpartikeln, die eine erhöhte Lagerstabilität aufweisen.

Überraschenderweise wurde gefunden, dass Mentholpartikel, erhältlich indem man 20 kg Mentholpartikel im Anschluss an die Formgebung
a) in einen Beutel (B) aus einer 0,12 mm dicken Polyethylen-Folie mit den Maßen Länge L(B) von 660 mm und Breite B(B) von 690 mm füllt, den Beutel verschließt;
   diesen Beutel in eine quaderförmige Kiste (K) aus zweiwelliger Wellpappe mit den Innenmaßen Länge L(K) von 385 mm, Breite B(K) von 320 mm und Höhe H(K) von 450 mm und einer Wellpappendicke von 6 mm verpackt
b) diese Kiste für 10 Tage bei 20 °C auf der Seite, die von L(K) und H(K) gebildet wird, lagert und
c) anschließend einmalig aus einer Höhe von 1,5 m planparallel mit der von L(K) und H(K) gebildeten Seite auf eine unelastische Oberfläche fallen lässt,
eine erhöhte Lagerstabilität aufweisen.

Ein weiterer Gegenstand der vorliegenden Erfindung betrifft lagerstabile Mentholpartikel, erhältlich, indem man 20 kg Mentholpartikel im Anschluss an die Formgebung
a) in einen Beutel (B) aus einer 0,12 mm dicken Polyethylen-Folie mit den Maßen Länge L(B) von 660 mm und Breite B(B) von 690 mm füllt, den Beutel verschließt,
   diesen Beutel in eine quaderförmige Kiste (K) aus zweiwelliger Wellpappe mit den Innenmaßen Länge L(K) von 385 mm, Breite B(K) von 320 mm und Höhe H(K) von 450 mm und einer Wellpappendicke von 6 mm verpackt
b) diese Kiste für 10 Tage bei 20 °C auf der Seite, die von L(K) und H(K) gebildet wird, lagert und
c) anschließend einmalig aus einer Höhe von 1,5 m planparallel mit der von L(K) und H(K) gebildeten Seite auf eine unelastische Oberfläche fallen lässt,
wobei die Lagerstabilität L = [(Z₁-Z₂)/Z₁], kleiner gleich 0,25, insbesondere kleiner gleich 0,2, vorzugsweise kleiner gleich 0,1 beträgt, wobei Z₁ die Anzahl der Partikel nach der Formgebung und Z₂ die Anzahl der Partikel 20 Wochen nach der Formgebung bedeuten.

Als Maß für die Lagerstabilität L wird die Anzahl der Partikel direkt im Anschluss an die Formgebung sowie nach 20 Wochen bestimmt.

Die Lagerstabilität im Sinne der vorliegenden Erfindung ist definiert als L = [(Z₁-Z₂)/Z₁], wobei Z₁ die Anzahl der Partikel nach der Formgebung und Z₂ die Anzahl der Partikel 20 Wochen nach der Formgebung bedeuten.

Zur Bestimmung der Anzahl der Partikel Z₁ wird eine definierte Masse von Partikeln (z.B. 100g) nach der Formgebung eingewogen und ausgezählt. Zur Bestimmung der Anzahl der Partikel Z₂ wird eine gleichgroße Masse von Partikeln (z.B. 100g) 20 Wochen nach der Formgebung eingewogen und ausgezählt. Die Anzahl der Partikel kann beispielsweise mikroskopisch bestimmt werden.

Üblicherweise werden die Partikel in den 20 Wochen von Formgebung bis zu Bestimmung der Partikel bei Temperaturen von 0 bis 30°C insbesondere von 20 bis 25°C, insbesondere bei 20°C gehalten.

In einer bevorzugten Ausführungsform der Erfindung beträgt die Lagerstabilität L kleiner gleich 0,2; insbesondere kleiner gleich 0,1, insbesondere kleiner gleich 0,05; vorzugsweise kleiner gleich 0,01; insbesondere kleiner gleich 0,001.

Auch eine Zunahme der Partikelzahl, z.B. als Folge einer durchgeführten Stabilisierungsmaßnahme, ist von der vorliegenden Erfindung umfasst.

In einer weiteren Ausführungsform der Erfindung liegt die Lagerstabilität im Bereich von -0,25 bis 0,25; insbesondere von - 0,2 bis 0,2, vorzugsweise von - 0,1 bis 0,1, insbesondere von - 0,05 bis 0,05, vorzugsweise von - 0,001 bis 0,001.

Vorzugsweise ist die Anzahl der Partikel 20 Wochen nach der Formgebung identisch zur Partikelzahl nach der Formgebung, in diesem Fall ist die Lagerstabilität L = 0.

In einer Ausführungsform der Erfindung liegt die Lagerstabilität L im Bereich von 0 bis 0,25; insbesondere im Bereich von 0 bis 0,2; insbesondere von 0 bis 0,1; insbesondere von 0 bis 0,05; insbesondere von 0 bis 0,01; insbesondere von 0 bis 0,001.

Als lagerstabile Mentholpartikel eignen sich alle vorgenannten Mentholpartikel.

Eine bevorzugte Ausführungsform der Erfindung betrifft lagerstabile Mentholpartikel, die eine Größe im Bereich von 1 bis 35 mm, insbesondere von 4 bis 35 mm, insbesondere von 5 bis 30 mm, vorzugsweise von 10 bis 25, vorzugsweise von 12 bis 24, insbesondere 15 bis 20 mm aufweisen.

Eine bevorzugte Ausführungsform der Erfindung betrifft lagerstabile Mentholpartikel, die eine Größe im Bereich von 4 bis 35 mm, insbesondere im Bereich von 5 bis 30 mm, vorzugsweise von 10 bis 25, vorzugsweise im Bereich von 12 bis 24, insbesondere 15 bis 20 mm aufweisen und deren Anteil an Mentholpartikeln mit einer Größe von kleiner 4 mm weniger als 5 Gew.-%, vorzugsweise als 2 Gew.-% und insbesondere weniger als 1 Gew.-%, ganz bevorzugt weniger als 0,5 Gew.-%, besonders bevorzugt weniger als 0,1 Gew.-% beträgt.

Besonders bevorzugt sind lagerstabile Mentholpartikel, in denen mehr als 80 Gew.-%, insbesondere mehr als 90, insbesondere mehr als 99,5, vorzugsweise mehr als 99,7 Gew.-%, Menthol, insbesondere L-Menthol, bezogen auf das Gesamtgewicht des Partikels vorliegt.

Besonders bevorzugt sind lagerstabile Mentholpartikel, in denen das Menthol als L-Menthol vorliegt.

Besonders bevorzugt sind lagerstabile Mentholpartikel, die in Form von Schuppen, Sphären oder Pastillen vorliegen, bevorzugt in Form von Schuppen.

Weiterhin bevorzugt sind lagerstabile Mentholpartikel, die in Form von Schuppen mit einer Dicke von 0,2 bis 15, bevorzugt von 4 bis 10 mm, insbesondere 6 bis 8 mm vorliegen.

Insbesondere bevorzugt sind lagerstabile Mentholpartikel Menthol in Form von Schuppen, die erhalten wurden durch Inkontaktbringen einer Menthol-Schmelze mit zwei voneinander beabstandeten gekühlten Oberflächen unter Erstarrung der Menthol-Schmelze zu Menthol, wobei man den Kontakt zwischen der erstarrenden Menthol-Schmelze und den gekühlten Oberflächen mindestens bis zum Abschluss der Erstarrung aufrechterhält.

Die so erhältlichen lagerstabilen Mentholpartikel eigen sich aufgrund ihrer Lagerstabilität insbesondere zur Herstellung von oder Verwendung in Gebrauchs- oder Verbrauchsgütern.

Ein weiterer Gegenstand der Erfindung betrifft daher die Verwendung erfindungsgemäße erhältlichen lagerstabilen Mentholpartikel zur Herstellung von oder in Gebrauchs- oder Verbrauchsgüter wie beispielsweise pharmazeutische oder kosmetische Zubereitungen, Nahrungsmittel, Hygiene- oder Reinigungsartikel, Süßwaren oder Tabakerzeugnisse.

### BEISPIELE

### Notensystem zur Bewertung der Verbackung:

0 = vollkommend frei rieselfähig, keine Verbackungen
1 = geringe Verbackungen, die sich leicht mit der Hand lösen lassen
2 = starke Verbackungen, die sich nur schwer oder gar nicht vereinzeln lassen

### I. Erfindungsgemäße Beispiele

### Beispiele 1A-3, 1B-1 und 10-2

Als Mentholpartikel wurden Mentholschuppen, herstellt gemäß den Beispielen A-3, B-1 und D-2 eingesetzt. Direkt im Anschluss an die Formgebung wurden jeweils 20 kg der Mentholpartikel in Beutel aus 0,12 mm dicker Polyethylenfolie mit den Abmessungen 660 mm [= L(B)] mal 690 mm [= B(B)] gefüllt, die Beutel wurden verschweißt und diese Beutel in Kisten verpackt. Als Kisten wurden Kisten aus zweiwelliger Wellpappe mit den Innenmaßen: Länge 385 mm [= L(K)], Breite 320 mm [= B(K)], Höhe 450 mm [= H(K)] und einer Wellpappendicke von 6 mm verwendet. Der Stapelstauchwiderstand (DIN 55440) F der Kiste beträgt 4500 N, das Gewicht liegt bei 0.8 kg, die Breite der Fabrikkante beträgt 40 mm. Es handelt sich um eine Faltkiste gemäß FEFCO 0201. Die Kisten wurden mittels Klebeband verschlossen und auf der durch L(K) und H(K) gebildeten Fläche bei 20 °C für 7 Tage gelagert. Danach wurden die Kisten aus einer Höhe von 1,5 m auf den Boden (Betonboden) fallen gelassen. Dazu wurden die Kisten mit Hilfe des Transportarms eines Fallroboters auf eine Höhe von 1,5 m gehoben, dann erfolgte der freie Fall auf den Betonboden. Die Aufprallfläche der Kiste ist die durch L(K) und H(K) gebildete Fläche der Kiste.

Die so behandelten Kisten wurden direkt nach dem Eintrag der mechanischen Energie sowie 18 Wochen (Lagerung bei 20°C) nach dem Eintrag der mechanischen Energie geöffnet und der Verbackungsgrad anhand der Notenskala (s.o.) bewertet: Für alle 3 Beispiele 1A-3, 1B-1 sowie 1D-2 wurde sowohl direkt nach dem Eintrag der mechanischen Energie als auch nach 18 Wochen der Verbackungsgrad der Mentholpartikel mit "0" bewertet.

### Beispiele 2A-3, 2B-1 und 2D-2

Beispiele 1A-3, 1B-1 und 1D-2 wurden wiederholt, allerdings erfolgte die Lagerung vor dem Eintrag der mechanischen Energie für 14 Tage.

Die so behandelten Kisten wurden direkt nach dem Eintrag der mechanischen Energie sowie 17 und 18 Wochen (Lagerung bei 20°C) nach dem Eintrag der mechanischen Energie geöffnet und der Verbackungsgrad anhand der Notenskala (s.o.) bewertet: Für alle 3 Beispiele 2A-3, 2B-1 sowie 2D-2 wurde sowohl direkt nach dem Eintrag der mechanischen Energie als auch nach 17 und 18 Wochen der Verbackungsgrad der Mentholpartikel mit "0" bewertet.

### Beispiele 3A-3, 3B-1 und 3D-2

Beispiele 1A-3, 1B-1 und 1D-2 wurden wiederholt, allerdings erfolgte die Lagerung vor dem Eintrag der mechanischen Energie für 21 Tage.

Die so behandelten Kisten wurden direkt nach dem Eintrag der mechanischen Energie sowie 16, 17 und 18 Wochen (Lagerung bei 20°C) nach dem Eintrag der mechanischen Energie geöffnet und der Verbackungsgrad anhand der Notenskala (s.o.) bewertet: Für alle 3 Beispiele 3A-3, 3B-1 sowie 3D-2 wurde sowohl direkt nach dem Eintrag der mechanischen Energie als auch nach 16, 17 und 18 Wochen der Verbackungsgrad der Mentholpartikel mit "0" bewertet.

### Beispiele 4A-3, 4B-1 und 4D-2

Beispiele 2A-3, 2B-1 und 2D-2 wurden wiederholt, allerdings wurden die Kisten aus einer Höhe von 1,0 m fallen gelassen.

Die so behandelten Kisten wurden direkt nach dem Eintrag der mechanischen Energie sowie 18 Wochen (Lagerung bei 20°C) nach dem Eintrag der mechanischen Energie geöffnet und der Verbackungsgrad anhand der Notenskala (s.o.) bewertet: Für alle 3 Beispiele 4A-3, 4B-1 sowie 4D-2 wurde sowohl direkt nach dem Eintrag der mechanischen Energie als auch nach 18 Wochen der Verbackungsgrad der Mentholpartikel mit "0" bewertet.

### Beispiele 5A-3, 5B-1 und 5D-2

Beispiele 2A-3, 2B-1 und 2D-2 wurden wiederholt, allerdings wurden die Kisten aus einer Höhe von 2,0 m fallen gelassen.

Die so behandelten Kisten wurden direkt nach dem Eintrag der mechanischen Energie sowie 18 Wochen (Lagerung bei 20°C) nach dem Eintrag der mechanischen Energie geöffnet und der Verbackungsgrad anhand der Notenskala (s.o.) bewertet: Für alle 3 Beispiele 5A-3, 5B-1 sowie 5D-2 wurde sowohl direkt nach dem Eintrag der mechanischen Energie als auch nach 18 Wochen der Verbackungsgrad der Mentholpartikel mit "0" bewertet.

### Beispiele 6A-3, 6B-1 und 6D-2

Beispiele 2A-3, 2B-1 und 2D-2 wurden wiederholt, allerdings wurden jeweils 10 kg der Mentholpartikel direkt im Anschluss an die Formgebung in Beutel gefüllt.

Die so behandelten Kisten wurden direkt nach dem Eintrag der mechanischen Energie sowie 18 Wochen (Lagerung bei 20°C) nach dem Eintrag der mechanischen Energie geöffnet und der Verbackungsgrad anhand der Notenskala (s.o.) bewertet: Für alle 3 Beispiele 6A-3, 6B-1 sowie 6D-2 wurde sowohl direkt nach dem Eintrag der mechanischen Energie als auch nach 18 Wochen der Verbackungsgrad der Mentholpartikel mit "0" bewertet.

### II. Vergleichsbeispiele

### Beispiele V1A-3, V1B-1 und V1D-2 - Mentholpartikel ohne Energie Eintrag

Als Mentholpartikel wurden Mentholschuppen, herstellt gemäß den Beispielen A-3, B-1 und D-2 eingesetzt. Direkt im Anschluss an die Formgebung wurden jeweils 20 kg der Mentholpartikel in Beutel aus 0,12 mm dicker Polyethylenfolie mit den Abmessungen 660 mm [= L(B)] mal 690 mm [= B(B)] gefüllt, die Beutel wurden verschweißt und diese Beutel in Kisten verpackt. Als Kisten wurden Kisten aus zweiwelliger Wellpappe mit den Innenmaßen: Länge 385 mm [= L(K)], Breite 320 mm [= B(K)], Höhe 450 mm [= H(K)] und einer Wellpappendicke von 6 mm verwendet. Der Stapelstauchwiderstand (DIN 55440) F der Kiste beträgt 4500 N, das Gewicht liegt bei 0.8 kg, die Breite der Fabrikkante beträgt 40 mm. Es handelt sich um eine Faltkiste gemäß FEFCO 0201. Die Kisten wurden mittels Klebeband verschlossen und auf der durch L(K) und H(K) gebildeten Fläche bei 20 °C gelagert.

Die Kisten wurden 18 bzw. 19 Wochen nach ihrer Befüllung geöffnet und der Verbackungsgrad anhand der Notenskala (s.o.) bewertet: für alle 3 Vergleichsbeispiele V1A-3, V1B-1 sowie V1D-2 wurde der Verbackungsgrad der Mentholpartikel sowohl nach 18 als auch nach 19 Wochen mit "2" bewertet.

### Beispiele V2A-3, V2B-1 und V2D-2 - Mentholpartikel mit Energie Eintrag direkt nach der Formgebung

Als Mentholpartikel wurden Mentholschuppen, herstellt gemäß den Beispielen A-3, B-1 und D-2 eingesetzt. Direkt im Anschluss an die Formgebung wurden jeweils 20 kg der Mentholpartikel in Beutel aus 0,12 mm dicker Polyethylenfolie mit den Abmessungen 660 mm [= L(B)] mal 690 mm [= B(B)] gefüllt, die Beutel wurden verschweißt und diese Beutel in Kisten verpackt. Als Kisten wurden Kisten aus zweiwelliger Wellpappe mit den Innenmaßen: Länge 385 mm [= L(K)], Breite 320 mm [= B(K)], Höhe 450 mm [= H(K)] und einer Wellpappendicke von 6 mm verwendet. Der Stapelstauchwiderstand (DIN 55440) F der Kiste beträgt 4500 N, das Gewicht liegt bei 0.8 kg, die Breite der Fabrikkante beträgt 40 mm. Es handelt sich um eine Faltkiste gemäß FEFCO 0201. Die Kisten wurden mittels Klebeband verschlossen und auf der durch L(K) und H(K) gebildeten Fläche bei 20 °C für 2 Stunden gelagert. Danach wurden die Kisten aus einer Höhe von 1,5 m auf den Boden (Betonboden) fallen gelassen. Dazu wurden die Kisten mit Hilfe des Transportarms eines Fallroboters auf eine Höhe von 1,5 m gehoben, dann erfolgte der freie Fall auf den Betonboden. Die Aufprallfläche der Kiste ist die durch L(K) und H(K) gebildete Fläche der Kiste.

Die so behandelten Kisten wurden direkt nach dem Eintrag der mechanischen Energie sowie 18 bzw. 19 Wochen (Lagerung bei 20°C) nach dem Eintrag der mechanischen Energie geöffnet und der Verbackungsgrad anhand der Notenskala (s.o.) bewertet: für alle 3 Vergleichsbeispiele V2A-3, V2B-1 sowie V2D-2 wurde direkt nach dem Eintrag der mechanischen Energie die Verbackung mit "0" bewertet, sowohl nach 18 als auch nach 19 Wochen wurde der Verbackungsgrad der Mentholpartikel mit "2" bewertet.

D.h. der erfindungsgemäße mechanische Energieeintrag führt zu einer Abnahme der Verbackung, diese hält jedoch nur an, wenn vor dem Eintrag der mechanischen Energie eine erfindungsgemäße Lagerung stattgefunden hat.

### III. Beispiele für Mentholpartikel, die im erfindungsgemäßen Verfahren eingesetzt werden können

### Beispiel A-1

Eine auf 50°C temperierte Schmelze von L-Menthol wurde in einen auf 10°C temperierten Kratzkühler eingetragen. Mittels des Kratzkühlers wurden Menthol Impfkristalle erzeugt, der Gehalt an Impfkristallen wurde mittels Messung der Dichte bestimmt und auf 20 Gew.-% eingestellt. Die so erhaltene Suspension aus geschmolzenem Menthol und 20 Gew.-% Menthol Impfkristallen wurde ausgetragen und über eine temperierte Rohrleitung auf ein von beiden Seiten auf 15°C temperiertes Doppelkühlband aufgebracht, der Bandabstand wurde auf 4 mm eingestellt. Nach 240 s Laufzeit wurde am Ende des Bandes ein 4 mm dicker durchkristallisierter Film von L- Menthol gewonnen, der mit einem Brechwerk in Schuppen mit einer Kantenlänge von 5 bis 25 mm zerkleinert wurde. Man erhielt auf beiden Seiten glänzend erscheinende Schuppen mit einer mittlere Dicke von 4 mm. Die so erhaltenen Schuppen wiesen eine Kantenlänge im Bereich von 5 bis 25 mm auf, die Mehrzahl der Schuppen wiesen eine Kantenläge im Bereich von 10 bis 16 mm auf.

### Beispiel A-2

Eine auf 50°C temperierte Schmelze von L-Menthol wurde in einen auf 10°C temperierten Kratzkühler eingetragen. Mittels des Kratzkühlers wurden Menthol Impfkristalle erzeugt, der Gehalt an Impfkristallen wurde mittels Messung der Dichte bestimmt und auf 20 Gew.-% eingestellt. Die so erhaltene Suspension aus geschmolzenem Menthol und 20 Gew.-% Menthol Impfkristallen wurde ausgetragen und über eine temperierte Rohrleitung auf ein von beiden Seiten auf 15°C temperiertes Doppelkühlband aufgebracht, der Bandabstand wurde auf 6 mm eingestellt. Nach 240 s Laufzeit wurde am Ende des Bandes ein 6 mm dicker durchkristallisierter Film von L- Menthol gewonnen, der mit einem Brechwerk in Schuppen mit einer Kantenlänge von 5 bis 25 mm zerkleinert wurde. Man erhielt auf beiden Seiten glänzend erscheinende Schuppen mit einer mittleren Dicke von 6 mm. Die so erhaltenen Schuppen wiesen eine Kantenlänge im Bereich von 5 bis 25 mm auf, die Mehrzahl der Schuppen wiesen eine Kantenläge im Bereich von 10 bis 16 mm auf.

### Beispiel A-3

Eine auf 50°C temperierte Schmelze von L-Menthol wurde in einen auf 10°C temperierten Kratzkühler eingetragen. Mittels des Kratzkühlers wurden Menthol Impfkristalle erzeugt, der Gehalt an Impfkristallen wurde mittels Messung der Dichte bestimmt und auf 20 Gew.-% eingestellt. Die so erhaltene Suspension aus geschmolzenem Menthol und 20 Gew.-% Menthol Impfkristallen wurde ausgetragen und über eine temperierte Rohrleitung auf ein von beiden Seiten auf 15°C temperiertes Doppelkühlband aufgebracht, der Bandabstand wurde auf 8 mm eingestellt. Nach 240 s Laufzeit wurde am Ende des Bandes ein 8 mm dicker durchkristallisierter Film von L- Menthol gewonnen, der mit einem Brechwerk in Schuppen mit einer Kantenlänge von 5 bis 25 mm zerkleinert wurde. Man erhielt auf beiden Seiten glänzend erscheinende Schuppen mit einer mittleren Dicke von 8 mm. Die so erhaltenen Schuppen wiesen eine Kantenlänge im Bereich von 5 bis 25 mm auf, die Mehrzahl der Schuppen wiesen eine Kantenläge im Bereich von 10 bis 16 mm auf.

### Beispiel B-1

Eine auf 50°C temperierte Schmelze von L-Menthol wurde in einen auf 20 °C temperierten Kratzkühler eingetragen. Mittels des Kratzkühler wurden Menthol Impfkristalle erzeugt. Der Gehalt an Impfkristallen wurde mittels Messung der Dichte bestimmt und auf 10 Gew.-% eingestellt. Die so erhaltene Suspension aus geschmolzenem Menthol und 10 Gew.-% Menthol Impfkristallen wurde ausgetragen und über eine temperierte Rohrleitung auf ein von beiden Seiten auf 15°C temperiertes Doppelkühlband aufgebracht, der Bandabstand betrug 6 mm. Nach 240 bis 300s Laufzeit wurde am Ende des Bandes ein 6 mm dicker, durchkristallisierter Film von L-Menthol gewonnen. Durch eine Vorzerkleinerung mit einem Stiftbrecher und in einer sich daran anschließenden Zerkleinerung mittels eines Sieb-Zerkleinerers (Rotorfeingranulator Typ 250 D, Hersteller Alexanderwerk, mit einem Siebeinsatz von 12 x 24 mm) wurden Menthol-Schuppen der gewünschten Größe erhalten. Nach einer sich anschließenden Feinstoff-Abtrennung mittels eines 4 mm Siebs, wurden Menthol-Schuppen der folgenden Größenverteilung erhalten:

| | Dicke in mm | Länge in mm | Breite in mm |
|---|---|---|---|
| Mittelwert | 5,3 | 15,3 | 11,5 |
| MAX | 10,5 | 22,4 | 19,0 |
| MIN | 2,3 | 7,6 | 5,7 |

| | | | |
|---|---|---|---|
| Der Anteil an Menthol-Partikeln mit einer Größe von kleiner 4 mm betrug < 0,1 Gew.-%. | | | |

### Beispiel B-2

Eine auf 50°C temperierte Schmelze von L-Menthol wurde in einen auf 20 °C temperierten Kratzkühler eingetragen. Mittels des Kratzkühler wurden Menthol Impfkristalle erzeugt. Der Gehalt an Impfkristallen wurde mittels Messung der Dichte bestimmt und auf 10 Gew.-% eingestellt. Die so erhaltene Suspension aus geschmolzenem Menthol und 10 Gew.-% Menthol Impfkristallen wurde ausgetragen und über eine temperierte Rohrleitung auf ein von beiden Seiten auf 15°C temperiertes Doppelkühlband aufgebracht, der Bandabstand betrug 8 mm. Nach 240 bis 300s Laufzeit wurde am Ende des Bandes ein 8 mm dicker, durchkristallisierter Film von L-Menthol gewonnen. Durch eine Vorzerkleinerung mit einem Stiftbrecher und in einer sich daran anschließenden Zerkleinerung mittels eines Sieb-Zerkleinerers (Rotorfeingranulator Typ 250 D, Hersteller Alexanderwerk, mit einem Siebeinsatz von 12 x 24 mm) wurden Menthol-Schuppen der folgenden Größenverteilung erhalten.

| | Dicke in mm | Länge in mm | Breite in mm |
|---|---|---|---|
| Mittelwert | 7,2 | 15,2 | 10,5 |
| MAX | 14,2 | 21,4 | 18,5 |
| MIN | 0,3 | 0,54 | 0,2 |

| | | | |
|---|---|---|---|
| Der Anteil an Menthol-Partikel mit einer Größe von kleiner 4 mm betrug 12 Gew.-%. | | | |

### Beispiel B-3

Eine auf 50°C temperierte Schmelze von L-Menthol wurde in einen auf 20 °C temperierten Kratzkühler eingetragen. Mittels des Kratzkühler wurden Menthol Impfkristalle erzeugt. Der Gehalt an Impfkristallen wurde mittels Messung der Dichte bestimmt und auf 10 Gew.-% eingestellt. Die so erhaltene Suspension aus geschmolzenem Menthol und 10 Gew.-% Menthol Impfkristallen wurde ausgetragen und über eine temperierte Rohrleitung auf ein von beiden Seiten auf 15°C temperiertes Doppelkühlband aufgebracht, der Bandabstand betrug 9 mm. Nach 240 bis 300s Laufzeit wurde am Ende des Bandes ein 9 mm dicker, durchkristallisierter Film von L-Menthol gewonnen. Durch eine Vorzerkleinerung mit einem Stiftbrecher und in einer sich daran anschließenden Zerkleinerung mittels eines Sieb-Zerkleinerers (Rotorfeingranulator Typ 250 D, Hersteller Alexanderwerk, mit einem Siebeinsatz von 12 x 24 mm) wurden Menthol-Schuppen der gewünschten Größe erhalten. Nach einer sich anschließenden Feinstoff-Abtrennung mittels eines 4 mm Siebs, wurden Menthol-Schuppen der folgenden Größenverteilung erhalten:

| | Dicke in mm | Länge in mm | Breite in mm |
|---|---|---|---|
| Mittelwert | 8,9 | 15,5 | 12,0 |
| MAX | 17,2 | 21,0 | 19,3 |
| MIN | 4,2 | 7,4 | 5,7 |

| | | | |
|---|---|---|---|
| Der Anteil an Menthol-Partikeln mit einer Größe von kleiner 4 mm betrug < 0,1 Gew.-%. | | | |

### Beispiel C-1

Eine auf 50°C temperierte Schmelze von L-Menthol wurde in einen auf 15°C gekühlten Extruder (mit gleichläufiger Doppelschnecke und integrierter Pumpe zur Förderung der L-Mentholschmelze, Auslasstemperatur von 42°C) eingetragen, mittels des Extruders wurden Menthol Impfkristalle erzeugt. Der Gehalt an Impfkristallen wurde mittels Messung der Dichte bestimmt und auf 30 Gew.-% eingestellt. Die so erhaltene Suspension aus geschmolzenem Menthol und 30 Gew.-% Menthol Impfkristallen wurde ausgetragen und über eine temperierte Rohrleitung auf ein von beiden Seiten auf 15°C temperiertes Doppelkühlband aufgebracht, der Bandabstand betrug 6 mm. Nach 160s Laufzeit wurde am Ende des Bandes ein 6 mm dicker, durchkristallisierter Film von L- Menthol gewonnen. Durch eine Vorzerkleinerung mit einem Stiftbrecher und in einer sich daran anschließenden Zerkleinerung mittels eines Sieb-Zerkleinerers (Rotorfeingranulator Typ 250 D, Hersteller Alexanderwerk, mit einem Siebeinsatz von 12 x 24 mm) wurden Menthol-Schuppen der gewünschten Größe erhalten. Nach einer sich anschließenden Feinstoff-Abtrennung mittels eines 4 mm Siebs, wurden Menthol-Schuppen der folgenden Größenverteilung erhalten:

| | Dicke in mm | Länge in mm | Breite in mm |
|---|---|---|---|
| Mittelwert | 5,6 | 15,4 | 11,2 |
| MAX | 10,7 | 22,7 | 19,0 |
| MIN | 2,1 | 7,3 | 5,8 |

| | | | |
|---|---|---|---|
| Der Anteil an Menthol-Partikeln mit einer Größe von kleiner 4 mm betrug < 0,1 Gew.-%. | | | |

### Beispiel C-2

Eine auf 50°C temperierte Schmelze von L-Menthol wurde in einen auf 15°C gekühlten Extruder (mit gleichläufiger Doppelschnecke und integrierter Pumpe zur Förderung der L-Mentholschmelze, Auslasstemperatur von 42°C) eingetragen, mittels des Extruders wurden Menthol Impfkristalle erzeugt. Der Gehalt an Impfkristallen wurde mittels Messung der Dichte bestimmt und auf 30 Gew.-% eingestellt. Die so erhaltene Suspension aus geschmolzenem Menthol und 30 Gew.-% Menthol Impfkristallen wurde ausgetragen und über eine temperierte Rohrleitung auf ein von beiden Seiten auf 15°C temperiertes Doppelkühlband aufgebracht, der Bandabstand betrug 8 mm. Nach 240s Laufzeit wurde am Ende des Bandes ein 8 mm dicker, durchkristallisierter Film von L- Menthol gewonnen. Durch eine Vorzerkleinerung mit einem Stiftbrecher und in einer sich daran anschließenden Zerkleinerung mittels eines Sieb-Zerkleinerers (Rotorfeingranulator Typ 250 D, Hersteller Alexanderwerk, mit einem Siebeinsatz von 12 x 24 mm) wurden Menthol-Schuppen der gewünschten Größe erhalten. Nach einer sich anschließenden Feinstoff-Abtrennung mittels eines 4 mm Siebs, wurden Menthol-Schuppen der folgenden Größenverteilung erhalten:

| | Dicke in mm | Länge in mm | Breite in mm |
|---|---|---|---|
| Mittelwert | 7,6 | 15,2 | 10,3 |
| MAX | 14,3 | 20,4 | 19,5 |
| MIN | 3,7 | 7,5 | 5,2 |

| | | | |
|---|---|---|---|
| Der Anteil an Menthol-Partikeln mit einer Größe von kleiner 4 mm betrug < 0,1 Gew.-%. | | | |

### Beispiel D-1

Eine auf 50°C temperierte Schmelze von L-Menthol wurde in einen auf 20°C gekühlten Extruder (mit gleichläufiger Doppelschnecke und integrierter Pumpe zur Förderung der L-Mentholschmelze, Auslasstemperatur von 42°C) eingetragen, mittels des Extruders wurden Menthol Impfkristalle erzeugt. Der Gehalt an Impfkristallen wurde mittels Messung der Dichte bestimmt und auf 20 Gew.-% eingestellt. Die so erhaltene Suspension aus geschmolzenem Menthol und 20 Gew.-% Menthol Impfkristallen wurde ausgetragen und über eine temperierte Rohrleitung auf ein von beiden Seiten auf 15°C temperiertes Doppelkühlband aufgebracht, der Bandabstand betrug 7 mm. Nach 240s Laufzeit wurde am Ende des Bandes ein 7 mm dicker, durchkristallisierter Film von L- Menthol gewonnen. Durch eine Vorzerkleinerung mit einem Stiftbrecher und in einer sich daran anschließenden Zerkleinerung mittels eines Sieb-Zerkleinerers (Rotorfeingranulator Typ 250 D, Hersteller Alexanderwerk, mit einem Siebeinsatz von 12 x 24 mm) wurden Menthol-Schuppen der gewünschten Größe erhalten. Nach einer sich anschließenden Feinstoff-Abtrennung mittels eines 4 mm Siebs, wurden Menthol-Schuppen der folgenden Größenverteilung erhalten:

| | Dicke in mm | Länge in mm | Breite in mm |
|---|---|---|---|
| Mittelwert | 6,8 | 15,5 | 11,3 |
| MAX | 17,5 | 23,0 | 19,7 |
| MIN | 4,1 | 6,8 | 5,2 |

| | | | |
|---|---|---|---|
| Der Anteil an Menthol-Partikeln mit einer Größe von kleiner 4 mm betrug < 0,1 Gew.-%. | | | |

### Beispiel D-2

Eine auf 50°C temperierte Schmelze von L-Menthol wurde in einen auf 20°C gekühlten Extruder (mit gleichläufiger Doppelschnecke und integrierter Pumpe zur Förderung der L-Mentholschmelze, Auslasstemperatur von 42°C) eingetragen, mittels des Extruders wurden Menthol Impfkristalle erzeugt. Der Gehalt an Impfkristallen wurde mittels Messung der Dichte bestimmt und auf 20 Gew.-% eingestellt. Die so erhaltene Suspension aus geschmolzenem Menthol und 20 Gew.-% Menthol Impfkristallen wurde ausgetragen und über eine temperierte Rohrleitung auf ein von beiden Seiten auf 15°C temperiertes Doppelkühlband aufgebracht, der Bandabstand betrug 9 mm. Nach 240s Laufzeit wurde am Ende des Bandes ein 9 mm dicker, durchkristallisierter Film von L- Menthol gewonnen. Durch eine Vorzerkleinerung mit einem Stiftbrecher und in einer sich daran anschließenden Zerkleinerung mittels eines Sieb-Zerkleinerers (Rotorfeingranulator Typ 250 D, Hersteller Alexanderwerk, mit einem Siebeinsatz von 12 x 24 mm wurden Menthol-Schuppen der folgenden Größenverteilung erhalten.

| | Dicke in mm | Länge in mm | Breite in mm |
|---|---|---|---|
| Mittelwert | 8,8 | 15,2 | 11,0 |
| MAX | 17,5 | 21,3 | 19,7 |
| MIN | 0,3 | 0,5 | 0,2 |

| | | | |
|---|---|---|---|
| Der Anteil an Menthol-Partikeln mit einer Größe von kleiner 4 mm betrug 13 Gew.-%. | | | |

## Patentansprüche

1. Verfahren zur Herstellung von gegen Verbackung stabilisierten Mentholpartikeln, **dadurch gekennzeichnet, dass** man Mentholpartikel im Anschluss an die Formgebung für mindestens 7 Tage bei einer Temperatur von 0 bis 30°C lagert und anschließend den Mentholpartikeln mindestens so viel mechanische Energie zuführt, wie sie erhalten, wenn man
- 20 kg dieser Mentholpartikel
- in einen Beutel (B) aus einer 0,12 mm dicken Polyethylen-Folie mit den Maßen Länge L(B) von 660 mm und Breite B(B) von 690 mm füllt, den Beutel verschließt,
- diesen Beutel in eine quaderförmige Kiste (K) aus zweiwelliger Wellpappe mit den Innenmaßen Länge L(K) von 385 mm, Breite B(K) von 320 mm und Höhe H(K) von 450 mm und einer Wellpappendicke von 6 mm verpackt, und
- diese Kiste einmalig aus einer Höhe von 1,0 m planparallel mit der von L(K) und H(K) gebildeten Seite auf eine unelastische Oberfläche fallen lässt.

2. Verfahren nach Anspruch 1, **dadurch gekennzeichnet, dass** die Anzahl der Partikel am Ende des Verfahrens noch mindestens 50% der zu Beginn des Verfahrens eingesetzten Anzahl an Partikeln beträgt.

3. Verfahren nach einem der vorhergehenden Ansprüche, **dadurch gekennzeichnet, dass** mindestens 50 Gew.-% der gegen Verbackung stabilisierten Mentholpartikel dieselbe Form aufweisen wie die eingesetzten Mentholpartikel.

4. Verfahren nach einem der vorhergehenden Ansprüche, **dadurch gekennzeichnet, dass** mindestens 50 Gew.-% der gegen Verbackung stabilisierten Mentholpartikel dieselbe Größe aufweisen wie die eingesetzten Mentholpartikel.

5. Verfahren nach einem der vorgenannten Ansprüche, **dadurch gekennzeichnet, dass** Menthol in den Mentholpartikeln als L-Menthol vorliegt.

6. Verfahren nach einem der vorgenannten Ansprüche, **dadurch gekennzeichnet, dass** in den Mentholpartikeln mehr als 80 Gew.-%, insbesondere mehr als 90, insbesondere mehr als 99,5, vorzugsweise mehr als 99,7 Gew.-%, Menthol, insbesondere L-Menthol, bezogen auf das Gesamtgewicht des Partikels vorliegt.

7. Verfahren nach einem der vorgenannten Ansprüche, **dadurch gekennzeichnet, dass** man Mentholpartikel einer Größe im Bereich 1 bis 35 mm, insbesondere 4 bis 35 mm, insbesondere von 5 bis 30 mm, vorzugsweise von 10 bis 25, vorzugsweise von 12 bis 24, insbesondere 15 bis 20 mm einsetzt.

8. Verfahren nach einem der vorgenannten Ansprüche, **dadurch gekennzeichnet, dass** man Mentholpartikel in Form von Schuppen, Sphären oder Pastillen, bevorzugt in Form von Schuppen, einsetzt.

9. Verfahren nach einem der vorgenannten Ansprüche, **dadurch gekennzeichnet, dass** man Mentholpartikel in Form von Schuppen mit einer Dicke von 0,2 bis 15, bevorzugt von 4 bis 10 mm, insbesondere 6 bis 8 mm einsetzt.

10. Verfahren nach einem der vorgenannten Ansprüche, **dadurch gekennzeichnet, dass** man als Mentholpartikel Menthol in Form von Schuppen einsetzt, die erhalten wurden durch Inkontaktbringen einer Menthol-Schmelze mit zwei voneinander beabstandeten gekühlten Oberflächen unter Erstarrung der Menthol-Schmelze zu Menthol, wobei man den Kontakt zwischen der erstarrenden Menthol-Schmelze und den gekühlten Oberflächen mindestens bis zum Abschluss der Erstarrung aufrechterhält.

11. Verfahren nach Anspruch 10, **dadurch gekennzeichnet, dass** man die einzusetzende Menthol-Schmelze vor dem Inkontaktbringen mit den gekühlten Oberflächen mit 0,1 bis 50 Gew.-%, insbesondere mit 1 bis 40, insbesondere 5 bis 35, vorzugsweise mit 10 bis 30 Gew.-% Impfkristallen von Menthol versetzt.

12. Verfahren nach Anspruch 11, **dadurch gekennzeichnet, dass** die Impfkristalle durch Behandlung der einzusetzenden Menthol-Schmelze in einem Kratzkühler oder einem Extruder gebildet werden.

13. Verfahren nach einem der Ansprüche 10 bis 12 **dadurch gekennzeichnet, dass** die beiden gekühlten Oberflächen eine planparallele Ausrichtung mit einem Abstand von 0,2 bis 15, vorzugsweise 2 bis 10, insbesondere 3 bis 9, insbesondere 5 bis 8 mm insbesondere 6 bis 8 mm zueinander aufweisen.

14. Gegen Verbackung stabilisierte Mentholpartikel, erhältlich nach einem Verfahren nach einem der Ansprüche 1 bis 13.

15. Lagerstabile Mentholpartikel, erhältlich indem man 20 kg Mentholpartikel im Anschluss an die Formgebung
a) in einen Beutel (B) aus einer 0,12 mm dicken Polyethylen-Folie mit den Maßen Länge L(B) von 660 mm und Breite B(B) von 690 mm füllt, den Beutel verschließt; diesen Beutel in eine quaderförmige Kiste (K) aus zweiwelliger Wellpappe mit den Innenma-ßen Länge L(K) von 385 mm, Breite B(K) von 320 mm und Höhe H(K) von 450 mm und einer Wellpappendicke von 6 mm verpackt;
a) diese Kiste für 10 Tage bei 20 °C auf der Seite, die von L(K) und H(K) gebildet wird, lagert und
b) anschließend einmalig aus einer Höhe von 1,5 m planparallel mit der von L(K) und H(K) gebildeten Seite auf eine unelastische Oberfläche fallen lässt,
wobei die Lagerstabilität L = [(Z₁-Z₂)/Z₁], kleiner gleich 0,25, insbesondere kleiner gleich 0,2, vorzugsweise kleiner gleich 0,1 beträgt, wobei Z₁ die Anzahl der Partikel nach der Formgebung und Z₂ die Anzahl der Partikel 20 Wochen nach der Formgebung bedeuten.

16. Mentholpartikel nach Anspruch 15, **dadurch gekennzeichnet, dass** sie wenigstens eines der folgenden a) bis e) aufweisen:
a) das Menthol liegt als L-Menthol vor;
b) in den Mentholpartikeln mehr als 80 Gew.-%, insbesondere mehr als 90, insbesondere mehr als 99,5, vorzugsweise mehr als 99,7 Gew.-%, Menthol, insbesondere L-Menthol, bezogen auf das Gesamtgewicht des Partikels vor;
c) die Mentholpartikel weisen eine Größe im Bereich 1 bis 35 mm, insbesondere 4 bis 35 mm, insbesondere von 5 bis 30 mm, vorzugsweise von 10 bis 25, vorzugsweise von 12 bis 24, insbesondere 15 bis 20 mm auf;
d) die Mentholpartikel liegen in Form von Schuppen, Sphären oder Pastillen vor, bevorzugt in Form von Schuppen;
e) die Mentholpartikel liegen in Form von Schuppen mit einer Dicke von 0,2 bis 15, bevorzugt von 4 bis 10 mm, insbesondere 6 bis 8 mm vor.

17. Verwendung von lagerstabilen Mentholpartikeln nach einem der Ansprüche 15 oder 16 zur Herstellung von oder in Gebrauchs- und Verbrauchsgütern.

## Claims

1. A method for producing menthol particles stabilized against caking, wherein menthol particles are stored at a temperature of 0 to 30°C for at least 7 days after forming and then the menthol particles are supplied with at least as much mechanical energy as they receive when
- 20 kg of these menthol particles
- are packed into a bag (B) made of a 0.12 mm thick polyethylene film and having the dimensions length L(B) of 660 mm and width B(B) of 690 mm, the bag is closed,
- this bag is packed in a cuboidal box (K) made of double-wall corrugated cardboard and having the internal dimensions length L(K) of 385 mm, width B(K) of 320 mm, and height H(K) of 450 mm and a corrugated cardboard thickness of 6 mm, and
- this box is allowed to fall once from a height of 1.0 m plane-parallel to the side formed by L(K) and H(K) onto an inelastic surface.

2. The method according to claim 1, wherein the number of particles at the end of the method is still at least 50% of the number of particles used at the start of the method.

3. The method according to either of the preceding claims, wherein at least 50% by weight of the menthol particles stabilized against caking have the same shape as the menthol particles used.

4. The method according to any of the preceding claims, wherein at least 50% by weight of the menthol particles stabilized against caking have the same size as the menthol particles used.

5. The method according to any of the preceding claims, wherein menthol is present in the menthol particles in the form of L-menthol.

6. The method according to any of the preceding claims, wherein the menthol particles contain more than 80% by weight, in particular more than 90%, in particular more than 99.5%, preferably more than 99.7% by weight, of menthol, in particular L-menthol, based on the total weight of the particles.

7. The method according to any of the preceding claims, wherein menthol particles of a size in the range 1 to 35 mm, in particular 4 to 35 mm, in particular from 5 to 30 mm, preferably from 10 to 25, preferably from 12 to 24, in particular 15 to 20 mm, are used.

8. The method according to any of the preceding claims, wherein menthol particles in the form of flakes, spheres or pellets, preferably in the form of flakes, are used.

9. The method according to any of the preceding claims, wherein menthol particles in the form of flakes having a thickness of 0.2 to 15, preferably from 4 to 10 mm, in particular 6 to 8 mm, are used.

10. The method according to any of the preceding claims, wherein the menthol particles used are menthol in the form of flakes obtained by contacting a menthol melt with two cooled surfaces spaced a distance apart, whereby the menthol melt solidifies to menthol, the contact between the solidifying menthol melt and the cooled surfaces being maintained at least until the completion of solidification.

11. The method according to claim 10, wherein 0.1% to 50% by weight, in particular 1% to 40%, in particular 5% to 35%, preferably 10% to 30% by weight of menthol seed crystals are added to the menthol melt to be used before the contacting with the cooled surfaces.

12. The method according to claim 11, wherein the seed crystals are formed by treating the menthol melt to be used in a scraped-surface exchanger or an extruder.

13. The method according to any of claims 10 to 12, wherein the two cooled surfaces are oriented plane-parallel to one another, a distance of 0.2 to 15, preferably 2 to 10, in particular 3 to 9, in particular 5 to 8 mm, in particular 6 to 8 mm, apart.

14. A menthol particle stabilized against caking obtainable by a method according to any of claims 1 to 13.

15. A storage-stable menthol particle obtainable when, after forming, 20 kg of menthol particles
a) are packed into a bag (B) made of a 0.12 mm thick polyethylene film and having the dimensions length L(B) of 660 mm and width B(B) of 690 mm, the bag is closed, and this bag is packed in a cuboidal box (K) made of double-wall corrugated cardboard and having the internal dimensions length L(K) of 385 mm, width B(K) of 320 mm, and height H(K) of 450 mm and a corrugated cardboard thickness of 6 mm;
a) this box is stored for 10 days at 20°C on the side formed by L(K) and H(K), and
b) is then allowed to fall once from a height of 1.5 m plane-parallel to the side formed by L(K) and H(K) onto an inelastic surface,
where the storage stability L = [(Z₁-Z₂)/Z₁] is less than or equal to 0.25, in particular less than or equal to 0.2, preferably less than or equal to 0.1, where Z₁ is the number of particles after forming and Z₂ is the number of particles 20 weeks after forming.

16. A menthol particle according to claim 15, wherein it exhibits at least one of the following a) to e):
a) the menthol is present in the form of L-menthol;
b) the menthol particles contain more than 80% by weight, in particular more than 90%, in particular more than 99.5%, preferably more than 99.7% by weight, of menthol, in particular L-menthol, based on the total weight of the particles;
c) the menthol particles have a size in the range 1 to 35 mm, in particular 4 to 35 mm, in particular from 5 to 30 mm, preferably from 10 to 25, preferably from 12 to 24, in particular 15 to 20 mm;
d) the menthol particles are present in the form of flakes, spheres or pellets, preferably in the form of flakes;
e) the menthol particles are present in the form of flakes having a thickness of 0.2 to 15, preferably from 4 to 10 mm, in particular 6 to 8 mm.

17. The use of storage-stable menthol particles according to either of claims 15 or 16 for the production of, or in, consumer goods and consumables.

## Revendications

1. Procédé de préparation de particules de menthol stabilisées contre l'agglomération, **caractérisé en ce qu'**on entrepose des particules de menthol à la suite du façonnage pendant au moins 7 jours à une température de 0 à 30°C et on fournit ensuite aux particules de menthol au moins la quantité d'énergie mécanique qu'elles reçoivent lorsque
- on introduit 20 kg de ces particules de menthol
- dans un sachet (B) constitué d'une feuille de polyéthylène de 0,12 mm d'épaisseur, présentant les dimensions longueur L(B) de 660 mm et largeur B(B) de 690 mm, on ferme le sachet,
- on emballe ce sachet dans une caisse parallélépipédique (K) en carton ondulé à double paroi présentant les dimensions internes longueur L(K) de 385 mm, largeur B(K) de 320 mm et hauteur H(K) de 450 mm et une épaisseur de carton ondulé de 6 mm et
- on laisse tomber cette caisse une fois d'une hauteur de 1,0 m dans un plan parallèle à la face formée par L(K) et H(K) sur une surface non élastique.

2. Procédé selon la revendication 1, **caractérisé en ce que** le nombre de particules à la fin du procédé représente encore au moins 50% du nombre des particules mis en œuvre au début du procédé.

3. Procédé selon l'une des revendications précédentes, **caractérisé en ce qu'**au moins 50% en poids des particules de menthol stabilisées contre l'agglomération présentent la même forme que celle des particules de menthol mises en œuvre.

4. Procédé selon l'une des revendications précédentes, **caractérisé en ce qu'**au moins 50% en poids des particules de menthol stabilisées contre l'agglomération présentent la même grosseur que celle des particules de menthol mises en œuvre.

5. Procédé selon l'une des revendications susmentionnées, **caractérisé en ce que** le menthol dans les particules de menthol se trouve sous forme de L-menthol.

6. Procédé selon l'une des revendications susmentionnées, **caractérisé en ce que** les particules de menthol présentent plus de 80% en poids, en particulier plus de 90, en particulier plus de 99,5, de préférence plus de 99,7% en poids, de menthol, en particulier de L-menthol, par rapport au poids total de la particule.

7. Procédé selon l'une des revendications susmentionnées, **caractérisé en ce qu'**on met en œuvre des particules de menthol d'une grosseur dans la plage de 1 à 35 mm, en particulier de 4 à 35 mm, en particulier de 5 à 30 mm, de préférence de 10 à 25, de préférence de 12 à 24, en particulier de 15 à 20 mm.

8. Procédé selon l'une des revendications susmentionnées, **caractérisé en ce qu'**on met en œuvre des particules de menthol sous la forme d'écailles, de sphères ou de pastilles, de préférence sous forme d'écailles.

9. Procédé selon l'une des revendications susmentionnées, **caractérisé en ce qu'**on met en œuvre des particules de menthol sous forme d'écailles présentant une épaisseur de 0,2 à 15, de préférence de 4 à 10 mm, en particulier de 6 à 8 mm.

10. Procédé selon l'une des revendications susmentionnées, **caractérisé en ce qu'**on met en oeuvre, en tant que particules de menthol, du menthol sous forme d'écailles qui ont été obtenues par mise en contact d'une masse fondue de menthol avec deux surfaces refroidies écartées l'une de l'autre avec solidification de la masse fondue de menthol en menthol, le contact entre la masse fondue de menthol en cours de solidification et les surfaces refroidies étant maintenu au moins jusqu'à la fin de la solidification.

11. Procédé selon la revendication 10, **caractérisé en ce qu'**on additionne la masse fondue de menthol à mettre en oeuvre, avant la mise en contact avec les surfaces refroidies, de 0,1 à 50% en poids, en particulier de 1 à 40, en particulier de 5 à 35, de préférence de 10 à 30% en poids de germes cristallins de menthol.

12. Procédé selon la revendication 11, **caractérisé en ce que** les germes cristallins sont formés par traitement de la masse fondue de menthol à mettre en œuvre dans un refroidisseur à surface raclée ou dans une extrudeuse.

13. Procédé selon l'une des revendications 10 à 12, **caractérisé en ce que** les deux surfaces refroidies présentent une orientation à plans parallèles à une distance de 0,2 à 15, de préférence de 2 à 10, en particulier de 3 à 9, en particulier de 5 à 8 mm, en particulier de 6 à 8 mm l'une de l'autre.

14. Particules de menthol stabilisées contre l'agglomération, pouvant être obtenues selon un procédé selon l'une des revendications 1 à 13.

15. Particules de menthol stables à l'entreposage, pouvant être obtenues en ce qu'on introduit 20 kg de particules de menthol, à la suite du façonnage,
a) dans un sachet (B) constitué d'une feuille de polyéthylène de 0,12 mm d'épaisseur, présentant les dimensions longueur L(B) de 660 mm et largeur B(B) de 690 mm, on ferme le sachet ; on emballe ce sachet dans une caisse parallélépipédique (K) en carton ondulé à double paroi présentant les dimensions internes longueur L(K) de 385 mm, largeur B(K) de 320 mm et hauteur H(K) de 450 mm et une épaisseur de carton ondulé de 6 mm et
a) on entrepose cette caisse pendant 10 jours à 20°C sur la face formée par L(K) et H(K) et
b) on laisse ensuite tomber cette caisse une fois d'une hauteur de 1,5 m dans un plan parallèle à la face formée par L(K) et H(K) sur une surface non élastique,
la stabilité à l'entreposage L = [(Z₁-Z₂)/Z₁] étant inférieure ou égale à 0,25, en particulier inférieure ou égale à 0,2, de préférence inférieure ou égale à 0,1, Z₁ signifiant le nombre de particules après le façonnage et Z₂ signifiant le nombre de particules 20 semaines après le façonnage.

16. Particules de menthol selon la revendication 15, **caractérisées en ce qu'**elles présentent au moins l'une des caractéristiques a) à e) suivantes :
a) le menthol se trouve sous forme de L-menthol ;
b) les particules de menthol présentent plus de 80% en poids, en particulier plus de 90, en particulier plus de 99,5, de préférence plus de 99,7% en poids, de menthol, en particulier de L-menthol, par rapport au poids total de la particule ;
c) les particules de menthol présentent une grosseur dans la plage de 1 à 35 mm, en particulier de 4 à 35 mm, en particulier de 5 à 30 mm, de préférence de 10 à 25, de préférence de 12 à 24, en particulier de 15 à 20 mm ;
d) les particules de menthol se trouvent sous forme d'écailles, de sphères ou de pastilles, de préférence sous forme d'écailles ;
e) les particules de menthol se trouvent sous forme d'écailles présentant une épaisseur de 0,2 à 15, de préférence de 4 à 10 mm, en particulier de 6 à 8 mm.

17. Utilisation de particules de menthol stables au stockage selon l'une quelconque des revendications 15 ou 16 pour la préparation de produits d'utilisation et de consommation ou l'incorporation dans ceux-ci.
